Europäisches Patentamt

European Patent Office

Office européen des brevets

(11) Publication number: **0 425 217 A2**

## EUROPEAN PATENT APPLICATION

(21) Application number: 90311556.6

(51) Int. Cl.⁵: **C12Q 1/68**

(22) Date of filing: 22.10.90

The applicant has subsequently filed a sequence listing and declared, that it includes no new matter.

(30) Priority: 23.10.89 US 425647
24.10.89 US 426387

(43) Date of publication of application:
02.05.91 Bulletin 91/18

(84) Designated Contracting States:
DE FR GB IT

(71) Applicant: **Ciba Corning Diagnostics Corp.
63 North Street
Medfield Massachusetts 02052(US)**

(72) Inventor: **Dimond, Randall
4409 Travis Terrace
Madison, WI(US)**
Inventor: **Ekenberg, Steven J.
1117 Perry Center Road
Mt. Horeb, WI(US)**
Inventor: **Hudson, Geoffrey R.
4150 Council Crest
Madison, WI(US)**
Inventor: **Jones, Christopher L.
10 Westbrook Circle
Madison, WI(US)**
Inventor: **Martinelli, Richard A.
71 Kilsyth Road
Brighton, MA(US)**
Inventor: **Monahan, John E.
942 West Street
Walpole, MA(US)**
Inventor: **Schumm, James W.
5483 Timber Ridge Trail
Madison, WI(US)**
Inventor: **Weisburg, William G.
3 Jillson Circle
Milford, MA(US)**

(74) Representative: **Froud, Clive et al
ELKINGTON AND FIFE Beacon House 113
Kingsway
London WC2B 6PP(GB)**

(54) **A hybridization assay for campylobacter rRNA.**

(57) Method of detecting the presence of one or more Campylobacter species in a test sample, and providing a plurality of oligonucleotide probes, each having a nucleotide sequence which is complementary to regions of Campylobacter 16S rRNA. The assay format consists of a solution hybridization of at least two labelled oligonucleotide probes one of which is specific to a region of a Campylobacter rRNA, and then the capture of the resulting hybrid complex, on a solid support for subsequent detection and quantitation. The assay has a quantitative sensitivity to detect $1.0 \times 10^4$ bacterium per ml of test sample. A test kit and apparatus suitable for the method are described. In an expanded variation of the method an assay and kit are described for detecting

the presence of one or more target nucleic acid sequences in a test sample.

## A HYBRIDIZATION ASSAY FOR CAMPYLOBACTER RRNA

The present invention generally relates to oligonucleotide probes and to immunochemical techniques, and of methods of using such probes in combination with immunochemical techniques for diagnostic and other applicable purposes. More specifically, the invention consists of a sandwich hybridization assay to detect the presence of one or more Campylobacter species; and in an expanded assay format, the detection of one or more target nucleic acid sequences in a test sample. The genus Campylobacter is now recognized as a major cause of acute bacterial enteritis. These spiral-shaped pathogens have been shown to colonize the surface of the intestinal track in humans; and although the disease is self-limiting, early antibiotic therapy reduces the duration of the disease and fecal excretion. Since the discovery that Campylobacter is a cause of enteritis, a large number of strains of this bacteria have been identified by biochemical methodologies; however, the mechanism by which these species cause disease is still unknown. Studies examining the determinants of virulence attributed to Campylobacter include colonization, adhesion, invasion, and cytotoxin and endotoxin production. Labigne-Roussel et al, 170 (4) p. 1704-1708, (1988). The major medically important enteric pathogens are: C. jejuni , C. coli , and C. laridis . The presence of Campylobacter and other micro-organisms in a test sample have generally been detected by culture techniques. Such techniques require that an appropriately prepared and viable sample be deposited on an appropriate microbiological media and placed under environmental conditions favorable to promote the growth of the organism. The resultant colonies are then examined for morphological and biochemical characteristics. In the early to mid 1970's, two new techniques were developed that promised, with further study and refinement, significant advances in medical care and specifically in the area of diagnostic assays. One of these technologies was based on the immunological system (development and use of monoclonal antibodies) which relies on, and is limited by the properties of certain classes of macromolecules. The other technology was based upon the manipulation of genetic materials, and includes products and processes for analysis of such moieties. The genetic approach encompasses all information contained in genetic materials, DNA and RNA. DNA consists of two complementary chains of nucleotides. Each chain in its denatured form has the ability to hybridize (anneal) to its complementary chain even in the presence of large numbers of unrelated nucleotide sequences. Nucleic acid hybridizations can be performed in a variety of combinations, DNA/DNA, DNA/RNA, RNA/RNA, and in solution or on a solid support. Traditional solid supports for hybridization assays have included nitrocellulose and chemically treated paper. Southern, J. Mol. Bio. 98 p. 503-517 (1975). Applications of such hybridization techniques involve the use of natural or synthesized labelled nucleic acid molecules (probes) which are specific to aberant nucleic acid molecules and to etiologic pathogens. Techniques for synthesizing nucleic acid hybridization probes consisting of sequences of deoxyribonucleotides or ribonucleotides are well known in the art. Typically, to construct a probe, a target DNA is isolated from a cell and denatured to form a single strand and copies of a portion of the strand are isolated or synthesized in a laboratory and then labelled. When exposed to a complementary strand of target DNA or RNA in a test sample, the labelled probe hybridizes its complementary target DNA or RNA sequence. Probes may be labelled using radioactive isotopes, fluorescing molecules, luminescent molecules, enzymes or immunochemical molecules. The label on the probe is then detected and the presence of target DNA or RNA of interest is thus detected. Rashtchian, EP - A - 232 085 (87300569.8), describes oligonucleotide sequences which hybridize Campylobacter 16S rRNA. A labelled (avidin) solid support (plastic tube) quantitation assay is disclosed; where a labelled (biotin) DNA probe hybridized to Campylobacter 16S rRNA is detected by measuring unbound label on the solid support (the amount of biotin bound to the tubes being estimated by absorbence after treatment of the the tubes with avidin). Goodson, EP - A - 238 332 (87302354.3) describes a colormetric liquid hybridization assay for detecting nucleic acid sequences using at least two labelled oligonucleotide probes; and then capture of the hybrid complex to a solid support (microtiter well) for separation. Methods are described for detecting a restriction site characteristic of sickle cell anemia and for detecting the pilus surface antigen for N. gonorrheae . Hogan et al, EP - A - 272 009 (87310363.4) describes a method for preparing oligonucleotide probes complementary to a variable region of rRNA, selected to be unique to non-viral organisms. Probes specific to Campylobacter 16S rRNA are disclosed. Rashtchian et al, U.S. Patent 4,785,086, describes DNA probes (900-1500 nucleotides) that are capable of hybridizing DNA of at least 80% of bacteria of in the species Campylobacter jejuni . Denatured bacterial DNA is immobilized on a binding support, the bacterial DNA is then hybridized by a labelled probe. Another method is cited which describes the use of unlabelled probes; where the contacting and detecting steps are performed by sandwich hybridization. Law et al, U.S. 4,745,181 describes the use of chemiluminescent labels in specific binding assays such as immunoassays or nucleic acid hybridization assays. A polysubstituted aryl acridinium ester is disclosed. Josephson, U.S.

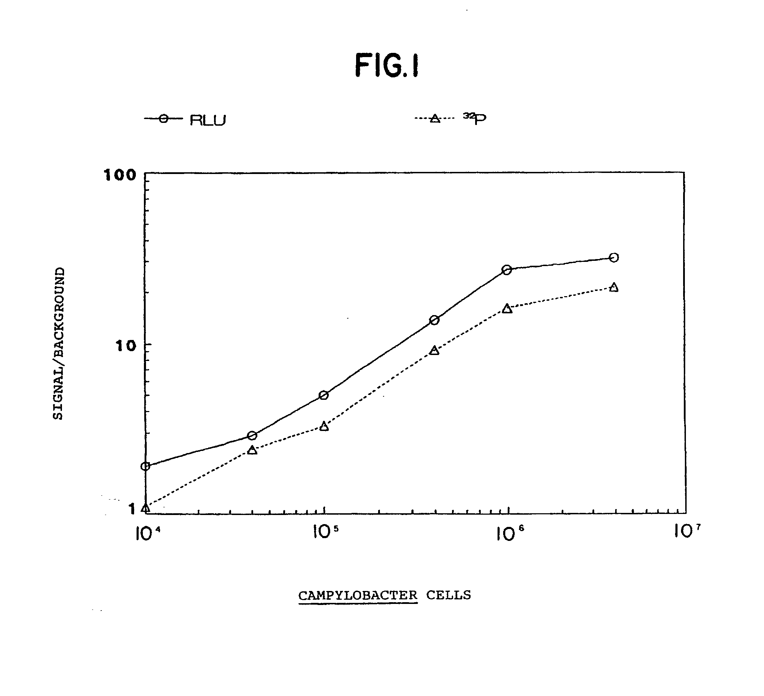

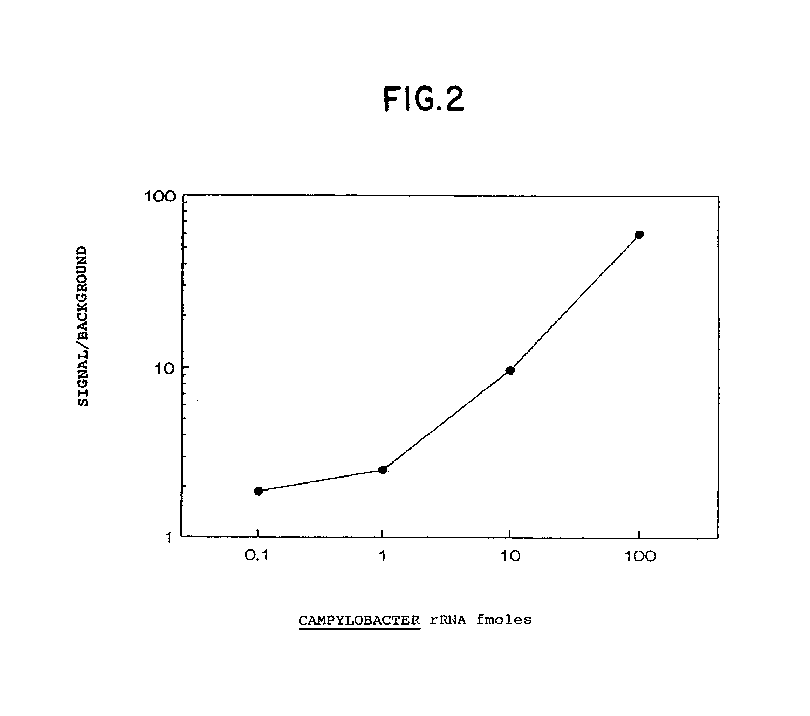

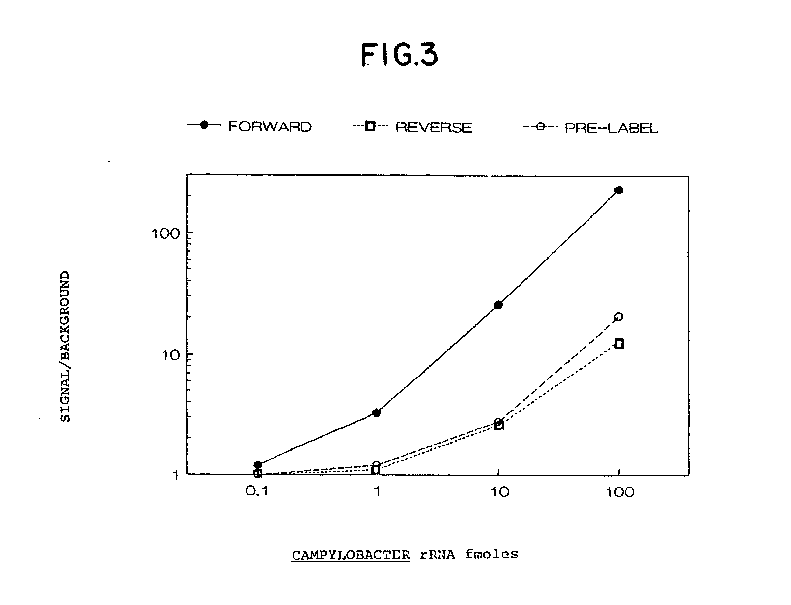

Patent 4,672,040, describes the use of magnetically responsive particles in nucleic acid hybridization assays. Nucleic acid coupled magnetic particles are dispersed in a reaction mixture containing molecules to be isolated, allowing hybridization and then separating the particles with the bound molecules from the reaction mixture. The hybridized molecule can then be separated from the magnetic particle. Hill et al, WO 86/5746 (PCT/GB86/00176) discloses the use of magnetic or magnetizable substance, coated with a material capable of attachment to single-stranded DNA or RNA for separation purposes. Following separation the material linked to the magnetic or magnetizable substance is contacted with a probe to detect the presence of the single-stranded material by hybridization. Rashtchian et al, Clin. Chem. 33/9 p. 1526-1530 (1987), describes an immunological capture method for nucleic acid hybrids and its application to nonradioactive labelled DNA probe assays. Synthetic DNA probes complementary to Campylobacter 16S rRNA were labelled with biotin and then hybridized to ribosomal RNA from lysates of bacterial cells. After hybridization, the hybrids were captured with immobilized anti-DNA:RNA antibody and the biotinylated probe was detected with streptavidin-horseradish peroxidase conjugate. The assay was optimized to detect 70,000 Campylobacter cells from a pure culture sample. Heller et al, EP-A-70687 (82303701.5), describes a light-emitting polynucleotide hybridization assay. A solid support method is described which comprises the steps of immobilizing a target single-stranded polynucleotide on a suitable support; contacting the immobilized sample with a labelled (peroxidase or iron porphrin derivative) single-stranded polynucleotide segments which are complementary to the target single-stranded polynucleotide; separating unhybridized single-stranded polynucleotide segments; exposing the immobilized hybrid to means for exciting the light label; and detecting the light response. Hansen, EP-A-139489 (84306513.7), describes a sandwich hybridization assay including the formation of a biotinylated nucleic acid probe bound to an avidin coated solid support and reacting the solid support bound probe with the hybridization product of an enzyme labelled nucleic acid and a target nucleic acid. Ranki et al., U.S. Patent 4,563,419 describes a competitive one-step sandwich hybridization assay for detection of target microbial nucleic acids. A first nucleic acid probe affixed to a solid support is hybridized with target nucleic acid sample and a labelled second nucleic acid probe. Following hybridization the label associated with the hybrid complex bound to the solid support is detected. Malcolm, WO 86/3782 (PCT/GB85/00591), describes a sandwich hybridization reaction (two overnight incubations) utilizing solid support (polymer beads) as a carrier for an immobilized nucleic acid fragment and a non-immobilized second labelled nucleic acid. Soderlund, UK Patent 2,169,403, recites a solution hybridization method for identification of nucleic acids. A detection (radio-label) nucleic acid probe and a capture nucleic acid probe are hybridized with the target nucleic acid sequence prior to capture to the solid support (affinity chromatography column). Snitman, WO 86/7387 (PCT/US86/01280), describes a solution hybridization assay including immobilizing the resultant hybrid complex on a solid support, and followed by a second hybridization of the target nucleic acid sequence. A modified method utilizes a distinct second probe during the first hybridization step in order to increase the capture to the solid support. Kohne, U.S. Patent 4,851,330, describes a method of detecting, identifying, and quantitating a group of non-viral organisms by hybridization assay. Other patents which may be considered to be of interest include (cited in alphabetic order): Chiswell, U.S. Patent 4,716,106 entitled: "Detecting Polynucleotide Sequences"; Daltagupta, U.S. Patent 4,670,380 entitled: "Assays Utilizing Labelled Nucleic Acid Probes"; Gingeras et al, WO 88/1302 (PCT/US87/01966) entitled: "Nucleic Acid Probe Assay Methods and Compositions"; Heller, EP - A - 269 764 (86118191.5), entitled: "Method for increasing the Sensitivity of Nucleic Acid Hybridization Assays"; Kourilsky et al, U.S. Patent 4,581,333 entitled: "Method of Detecting a Nucleic Acid or Reactant for the Application of this Method"; Miller, EP - A - 185 547 (85309224.5), entitled: "Polynucleotide Hybridization Assays Employing Catalyzed Luminescence"; Nogueira et al, U.S. Patent 4,801,530 entitled: "Nucleotide Hybridization Assay for Protozoan Parasites"; Rabboni et al, EP - A - 159 719 (85105130.0) entitled: "Hybridization Method for Detection of Genetic Materials"; Stabinsky, U.S. Patent 4,797,355 entitled: "Methods for Attaching Polynucleotides to Supports"; Taber et al, U.S. Patent 4,689,295 entitled: "Test for Salmonella". There remains still a need in the art for a timely simple and sensitive method for detecting one or more bacteria genera or species, which utilizes the rapid kinetics of liquid hybridization and also allows the hybridized products to be separated from unhybridized probes and debris of the test sample. The following terms, as used herein are defined as: 1. Bacteria: members of the phylogenetic group eubacteria, which is considered to be one of the three primary kingdoms. 2. Complementarity: a property conferred by the base sequence of single strand of DNA or RNA which may form a hybrid or a double-stranded DNA:DNA, RNA:RNA or DNA:RNA through hydrogen bonding between Watson-Crick base pairs on the respective strands. Adenine (A) usually complements Thymine (T) or Uracil (U), while Guanine (G) usually complements Cytosine (C). 3. Hybrid: the complex formed between two single stranded nucleic acid sequences by Watson-Crick base pairing or non-canonical base pairings between the complementary bases. 4. Hybridization: the process, environment and conditions under which at least two complementary strands of nucleic acids combine (anneal) to form a double-stranded molecule (hybrid). 5. Kit: a packaged combination of containers holding the necessary assay components for performing the sandwich hybridization method to detect the presence of one or more target nucleic acid sequences in a test sample. Apparatus, instrumentation devices and standard reagents necessary for performing the assay may or may not be a component(s) of the kit. 6. Liquid hybridization: refers to a hybridization of one or more nucleic acid probes and a target nucleic acid sequence in a liquid medium, without the presence of any solid support. 7. Mutually exclusive region: means that under hybridization conditions of the preferred embodiment the two probes should not compete for the same nucleotide base sequence on the target to the extent that hybridization of one probe prevents the hybridization of other probe(s). 8. Nucleic acid probe: a single-stranded nucleic acid sequence that will combine (anneal) with a complementary single-stranded target nucleic acid sequence to form a double-stranded molecule (hybrid). A nucleic acid probe may be an oligonucleotide. 9. Nucleotide: a subunit of nucleic acid consisting of a 5' phosphate group, a 5 carbon sugar and a nitrogen-containing base. In RNA the 5 carbon sugar is ribose. In DNA the 5 carbon sugar is a 2-deoxyribose. 10. Oligonucleotide: a nucleotide polymer generally about ten to fifty nucleotides in length. 11. Probe specificity: characteristic of a probe which describes its ability to distinguish between target and non-target nucleic acid sequences. Probe specificity may be absolute (i.e. probe able to distinguish between target organisms and non-target organisms), or it may be functional (i.e., probe able to distinguish between the target organism and any other organism normally present in a test sample). Many probe sequences may be adapted for use either broadly or narrowly depending upon the assay conditions of such use. 12. Sandwich immunoassay: involves coupling an antibody (monoclonal or polyclonal) directed to a first antigenic determinant to a solid support and exposing the solid support-coupled antibody to a test sample containing a substance bearing the first and a second antigenic determinant. This results in the removal of the antigenic substance from the sample by the formation of a primary antibody-antigen complex which is bound to the support. The complex is then exposed to a second labelled antibody directed toward a second antigenic determinant on the antigenic substance to create an antigen antibody sandwich which can be separated and detected. The sandwich assay may be modified to incorporate the use of other pairs of complementary molecules. 13. Target nucleic acid: refers to a segment of single-stranded polynucleotides having a nucleotide base sequence corresponding to a genetic element whose presence in a test sample is to be detected. 14. Test sample: refers to sample containing one or more target nucleic acids and which may be in purified or nonpurified form. Test samples may be obtained from any physiological or laboratory source, for example from cells, biological tissue extract, DNA or RNA (synthesized or natural) from any source including viruses, and the like. It is the primary object of the invention to provide an assay for detecting and quantitating the presence of one or more Campylobacter species in a test sample, and one which affords a more timely, specific, and sensitive methodology over culture techniques. Another object of the invention to provide a test kit for detecting and quantitating the presence of one or more genus or species of bacteria in a test sample. A further object of the invention to provide a plurality of oligonucleotide probes which are complementary and specific to Campylobacter 16S rRNA. It is another object of the invention is to provide an oligonucleotide probe assay which does not require the use of radioactive materials. It is still a further object of the invention to provide an assay which will allow quantitation of Campylobacter in a test sample having about 10,000 cells/ml of the bacterium. Still another object of the invention is to improve the detection of chemiluminescent labelled entities, and particularly chemiluminescent labelled oligonucleotide probes. Yet another object of the invention is to provide an assay for detecting the presence of one or more target nucleic acid sequences in a test sample, and one which affords a more timely, specific and sensitive methodology. In general, the invention consists of a sandwich hybridization assay for Campylobacter species. The assay is a two-step procedure, the first step involves a solution hybridization, which results in the formation of a hybrid complex comprising a target nucleic acid sequence and at least two labelled oligonucleotide probes, each probe being complementary and at least one probe being specific to a region of the target nucleic acid sequence. In the second step the hybrid complex is captured by a solid support. The label of at least one probe (i.e. detector probe) being used for the detection of the target nucleic acid sequence; and the label of at least one probe (i.e. capture probe) being used to bind to the solid support. Only target nucleic acid sequences hybridized with both the detector and capture probes, and bound to the solid support are detectable in the described assay format. The format of the assay may be expanded to incorporate a plurality of units of oligonucleotide probes, where each unit includes at least one capture probe and at least one detector probe. The capture and detector probes of each unit being complementary and at least one being specific to a region of the target nucleic acid sequence from a test sample. The detection and quantitation of one or more target nucleic acid sequences, for example one or more genus or species of bacteria, in a test sample is performed similarly to the described Campylobacter assay. The preferred assay format incorporates a chemiluminescent molecule

5

as the label of the detector probe; the chemiluminescent molecule being an acridinium ester. The chemiluminescent molecule reacts with appropriate reaction reagents to produce a light signal which enables the assay to provide a level of sensitivity to detect target nucleic acid sequences from a test sample which contains approximately $1 \times 10^4$ bacteria cells per ml of test sample. The preferred assay of this invention further incorporates the use of solid support, labels, and devices of the Magic$^R$ Lite assay system (Ciba Corning Diagnostics Corp.); however, other solid supports, including Sepharose 6B-CL, capture or detector labels, and devices can be employed. With such modifications, improvements in detection sensitivity can also be obtained in accordance with the principles of this invention. Referring to the accompanying drawings: Figure 1 illustrates the results of the comparative label assay for Campylobacter as described in Example 1. Figure 2 illustrates the results of a Magic$^R$ Lite sandwich hybridization assay for Campylobacter rRNA as described in Example 2. Figure 3 illustrates the results of a Magic$^R$ Lite sandwich hybridization assays varying the format of chemiluminescent labelling as described in Example 3. Oligonucleotide probes may be prepared synthetically, semisynthetically, by recombinant-DNA techniques, or from nucleic acids isolated from purified target nucleic acid sequence samples. Probes are also available from several sources, including Promega Corp., Madison, WI, U.S.A. The present invention incorporates methods for the synthetic preparation of DNA oligonucleotide probes for use in hybridization assays of Campylobacter rRNA. See Applied Biosystems Model 380B DNA Synthesizer Users Manual, Version 1.0, July, 1985. Applied Biosystems Model 381A DNA Synthesizer Users manual, Version 1.11, November, 1985. Beaucage et al, Tetrahedron letts . 22: 1859-1862 (1981). Matteucci and Caruthers, J. Am. Chem. Soc . 103: 3185-3191 (1981). Sinka et al., Tetrahedron Letts. 24: 5843-5846 (1983). These procedures may be utilized to prepare oligonucleotide probes for other target nucleic acid sequences including various genera or species of bacteria to be tested for in the expanded format of the assay. Table 1 lists thirteen oligonucleotide probes specific and complementary for Campylobacter 16S rRNA. The first column provides an assigned alphanumeric designation for each probe (assigned by Promega Corp.), the second column shows the location of the probe corresponding to E. coli 16S rRNA which they hybridize, the third column shows the base length of the probe, and the fourth column shows the sequence of the probe. It is noted that probes PM 78, PM 122 and PM 138 correspond to the same region of the E. coli 16S rRNA. The probes of Table 1 were tested for species specificity by a hybridization procedure. A slot blot hybridization procedure was used to assay the specificity of the probes on a variety of purified RNA samples isolated from Campylobacter (n = 133) and non-Campylobacter organisms (n = 73). See Kafatos et al, Nucl. Acid Res. 7(6) p. 1541-1552 (1970) and Tolsty et al, Cold Spring Harbor Laboratory (R.C. Schimke, ed.) p. 231-238 (1982). The RNA samples were isolated according to the method listed in Table 2. Table 3 provides detailed hybridization results for each probe and each strain of micro-organism analyzed. A zero (0) indicates lack of hybridization, a two (2) indicates strong hybridization, a one (1) indicates weak hybridization, and blank space indicates that no hybridization data was interpreted for that particular strain. Reference is made to the legend in Table 3. An abbreviated summary of the data contained in Table 3 is provided in Table 4. On the basis of analyzing the results of Table 3, at least two of said probes may be selected and incorporated for use in the Campylobacter assay of the preferred embodiment. For example, probe PM78 may be selected in one assay format based on its ability to detect C. fetus . In the same assay format probes PM 122 and PM 138 should not be selected as the second probe, since each may compete for the same 16S rRNA region as PM 78. It is understood that many of the same strains of micro-organisms analyzed for probe specificity in Table 3 will comprise targets for oligonucleotide probe units to be incorporated in the expanded assay format as described below. similar species specific testing would be required before incorporating such oligonucleotide probe-units into the expanded format. After the oligonucleotide probes are tested for species specificity, the ends of individual probes are modified by chemical groups capable of forming a stable complex with an analog of the said chemical group. See Smith et al, Nucl. Acid Res. 13(7) p. 2399-2411 (1986) and Haralambidls et al, Nucl. Acid Res. 15(12) p. 4857-4864 (1987). The chemical groups are chosen so as not to interfere with the hybridization step of the assay. In the described Examples and in the preferred embodiment at least one probe is labelled with one or more first support binding partner(s) and at least one probe is labelled with one or more detector molecule(s) by techniques, known in the art, including covalent bonding. The function of the support binding partners is to facilitate the capture (separation) of the hybrid complex to the solid support. The purpose and function of the solid support is described below. The hybrid complex comprises a target nucleic acid sequence annealed with at least two distinct oligonucleotide probes, with at least one of said probes having one or more first support binding partners bound thereto. The solid support in turn, includes one or more second support binding partners immobilized thereon, which have specific affinity to said first support binding partner. The high affinity of biotin for avidin or strepavidin is well suited for their use as support binding partners in the present invention. U.S. Patent 4,582,810, describes the formation of avidin-biotin bridges in a diagnostic composition with binding to solid support particles. Murasugi et al, DNA 3(3) p. 269-277 (1984) describes the use of biotin labelled oligonucleotides as hybridization probes. An immunoassay generally refers to a method of determining the presence or quantity of a substance in a test sample which method is based on the use of antibodies specific to that substance. The assay reaction requires the formation of an immunochemical complex between the antigenic substance (hapten) and its respective antibody, this occurs by simply incubating the antibody with a test sample containing the antigen. Either of the reactants of the immunochemical complex may be immobilized on a solid support as provided in one of the embodiments of this invention. If a hapten is used as the first support binding partner, such as dinitrophenol (DNP) of Example 1, described below, then an antibody (preferable monoclonal) to DNP, and specifically anti-DNP (5HI) in the preferred embodiment, will serve as the complementary second support binding partner. Numerous non-radioactive and radioactive labelling techniques, and detection protocols are known for detecting a hybridized complex. A detector molecule is typically chosen so that there is minimal interference with the base pairing (Watson-Crick) between the oligonucleotide probes and the target nucleic acid sequence. Examples of such molecules include radioactive, luminescent or fluorescent materials, enzymes which create luminescent, fluorescent, or colorometric products, and others as known in the art. In the preferred embodiment the detector molecule is a chemiluminescent molecule and more specifically an acridinium ester or a polysubstituted acridinium ester. The preparation and chemistry of polysubstituted aryl acridinium esters is described in U.S. Patent 4,745,181, and which is incorporated by reference. The light emission (signal) generated by the activation of the chemiluminescent molecule may be detected by a commercially available instrument, for example, a MLA instrument. (Ciba Corning Diagnostic Corp.) One or more detector molecules may be bound to an oligonucleotide probe in order to enhance detection sensitivity. It is a requirement of this information that no first binding partners be bound to the probe labelled with the detector molecules. At least one oligonticleotide probe labelled with one or more detector molecules comprise one component of the oligonucleotide probe unit in the expanded assay format of the present invention. Where a plurality of oligonucleotide probe units are incorporated into a desired assay format, more than one type of detector molecule per probe unit may be required to differentiate the signals or reactions generated on the activation of the detector molecules. If more than one detector molecule is employed, they may be detected sequentially in a manner as shown below in Example 1, or by use of chemiluminescent labels having expression signals at distinct wavelengths Alternatively, multiple detector molecules may be detected simultaneously. The labelled oligonucleotide probes may now be utilized in a hybridization assay for a target nucleic acid sequence. The test sample preparation requirements includes the lysis of the organisms of interest and the protection of the released RNA from the nucleases found in the sample. An optimal lysis procedure would lyse the desired enteric pathogens without requiring that every cell in the specimen lyse. Campylobacter cells were found to lyse in a Tris, EDTA buffer (pH 8-9) which contains SDS in the range of 0.05% to 0.5% at room temperature. Cells remain viable in the Tris, EDTA buffer in the absence of SDS. A lysis time of 1 to 10 minutes was acceptable. Shigella and Salmonella cells were more resistant to lysis than Campylobacter . Acceptable conditions for the quantitative lysis of these organisms required either a higher SDS concentration (0.25%) or a higher temperature. Lysis in 0.25% SDS in a Tris, EDTA buffer (pH 8-9) for 10 minutes at room temperature was the preferred procedure for cultured Shigella , Salmonella , and Campylobacter cells. This procedure, however, does not protect the released RNA in a stool specimen. In order to recover released RNA from a stool specimen other steps are required. The preferred method is to combine SDS lysis with a heat inactivation step, ( 75°C for 10 minutes) followed with filtration (LID/x (Genex) 25 um polyethylene). Other nuclease inhibitors were examined, but none other than the heat inactivation with SDS are required. For example, the inclusion of 5 to 10 mM VRC (vandyl ribonucleoside complex) with lysis at 65°C stabilizes the RNA as well as a lysis at 75°C without VRC. In the present invention each of the oligonucleotides are specific and complementary and at least one of which is specific to a target nucleic seqence suspected of being present in the test sample. The synthetic oligonucleotide probes may be both complementary and specific, for example, to any one of the variable regions of the rRNA, comprising the 5S, 16S or 23S rRNA. In the described method for detecting and quantitating the presence of Campylobacter species in a test sample, the oligonucleotide probes, as listed in Table 1, are both specific and complementary to mutually exclusive regions of Campylobacter 16S rRNA. Hybridization conditions necessary to accomplish annealing of the target nucleic acid sequence, if present, and at least two distinct oligonucleotide probes are determined by a number of variables, many of which may be adjusted to enhance the efficiency of the assay. The variables include: the nature of the labels attached to the probe, the sequence of the probe, the size (base pairs) of the probe, method of release/preparation of target nucleic acid sequence, and the duration and temperature of hybridization. It is also acknowledged that technical experience influences the efficiency of test assay. It is one aspect of this invention that these variables be reduced or made more uniform by providing a test kit which utilizes packaged assay components and the use of such kit components in combination with automated instrumentation (MLA system, Ciba Corning Diagnostics Corp.). Immobilization and separation of the hybrid complex from test sample debris and excess unhybridized probe is accomplished by use of a solid support. It is one of the advantages of a solid support that a plurality of binding partners can be immobilized thereon. Solid supports which have been utilized in hybridization assays include plastic tubes, microtiter wells, cross-linked dextran, porous silicate glass, magnetic particles coated with cellulose derivatives, nitrocellulose filter and other known materials which are inert to the assay components. In the preferred embodiment paramagnetic particles (PMP) are utilized as the solid support. The paramagnetic particles are of the type disclosed in U.S. Patent 4,554,088. The immobilization of the second support binding partner on the PMP may be accomplished by a number of techniques depending on the characteristics of the second support binding partner. In the case where the second support binding partner is an antibody, the antibody may be coupled to PMP by utilizing glutaraldehyde as a coupling agent. See Reichlin, Method of Enzy. , 70 p. 159-16S (1980). In the preferred embodiment the second support binding partner functions to capture the hybrid complex to the solid support. The complex is then separated from test sample debris and excess unhybridized probe by use of a magnetic field. (MLA rack, Ciba Corning Diagnostics Corp.) Alternate methods of separating the sandwich hybrid complex are described in the art, however, the MLA rack provides the advantage of processing a plurality of test samples simultaneously.

## EXAMPLE 1

Varying amounts of Campylobacter jejuni cells ($1 \times 10^4$ to $4 \times 10^6$) of a known source, ATCC #29428, were added to 100 ul of a 1:50 dilution of feces containing 50 mM Tris (Sigma Chemical Co.), pH 9.0, 0.6 M NaCl (Sigma Chemical Co.), 60 mM sodium citrate (Mallinckrodt), pH 7.5 and 0.05% SDS (Sigma Chemical Co.). The cells of the sample preparation were lysed by heating to 95° C for 5 minutes. The released rRNA was hybridized for 2.0 hrs. at 56°C with 0.9 picomoles (pmol) labelled oligonucleotide probe (DNP-PM78, DNP labelled at its 5′ end)) and 0.2 pmol labelled oligonucleotide probe (PM238), labelled with a chemiluminescent material (acridinium ester (AE)) at its 5′ terminus and $^{32}$P at its 3′ terminus. The nucleotide sequence of PM78 comprises: 5′-TCT GCC TCT CCC TCA CTC TAG ACT ATG AGT T-3′. The nucleotide sequence of PM238 comprises: 5′-GCC TTC GCA ATG GGT ATT CTT GGT GAT-3′. Following hybridization, 10 ul aliquots were added to 50 ug of paramagnetic particles (PMP) to which anti-DNP antibodies (mouse monoclonal) had been covalently attached. After 0.50 hrs. of incubation at room temperature (23° C), the bound sandwich hybrid was separated from excess unhybridized probes and test sample by separating the PMP in a Magic$^R$ Lite rack, (Ciba Corning Diagnostics Corp.) then the supernatant was removed. The PMP were washed twice with 0.6 M NaCl, 60 mM sodium citrate, 10 mM Tris, pH 7.0, 50 mM EDTA, (Sigma Chemical Co.), 0.1% bovine serum albumin (BSA) (Miles, fraction V) and 0.02% Tween-20 (Sigma Chemical Co.) and then resuspended in 100 ul of distilled water. It is noted that the solid support will also capture unhybridized probes labelled with the capture molecule but will not capture unhybridized probes labelled with the detector molecule. The bound sandwich hybrids were detected by a chemiluminescent reaction expressed in relative light units (RLU) in a Magic$^R$ Lite analyzer, (Ciba Corning Diagnostics Corp.) using modified reaction reagents (Reagent 1 comprising 1.0 N HNO$_3$ in a 0.5% solution of H$_2$O$_2$, Reagent 2 comprising 2.5N NaOH in a 0.5% solution of surfactant (Arquad)) (See also Example 6) (Ciba Corning Diagnostics Corp.) as well as liquid scintillation counting (atom light liguid scintillation cocktail, New England Nuclear) of the samples. Campylobacter rRNA was detected above background at cell counts in the order of 10,000 Campylobacter cells in a test sample. Figure 1 provides a comparison of the two labels relative to the concentration of Campylobacter cells in a test sample. As a control, purified E. coli rRNA (Pharmacia) were hybridized, captured and detected in the same manner (except in the absence of feces) and did not give light signals above background signal. The background signal was the chemiluminescent reaction observed (RLU expressed) from samples treated in the same manner but whose binding to be labelled PMP was blocked by the addition of beta-analine DNP. The conversion of the signal to cell counts was calculated by the following formula: The concentration of Campylobacter jejuni cells in suspension was determined by quantitative culture or fluorometric assay with a bibenzimidazole dye (Hoechst 33258). Cell suspensions were diluted $10^6$ and $10^8$ fold in ice cold sterile water. samples (10 and 100 ul) of dilutions were innoculated onto Brucella agar with 5% horse blood or Trypsoy agar with 10% sheep blood. Plates were incubated at 37° C in a microaerophilic environment (5% O$_2$, 10% CO$_2$ and 85% N$_2$). Colonies were counted after 48 hours of incubation. Fluorometric determination of the DNA concentration of Campylobacter jejuni suspensions was made using Hoechst 33258 dye. Concentration of DNA in the suspension was determined by incubation of the dell suspension and dye for 0.50 hrs. at room temperature (23°C) in the dark. Fluorescent readings were compared to a lambda DNA standard curve. Calculation of the number of cells/$\mu$l was based upon the genome size of C. jejuni :

1) $$\frac{2.2 \times 10^9 \text{ g/mole}}{6.02 \times 10^{23} \text{ molecules/mole}} = 3.65 \times 10^{-15} \text{ g DNA/cell}$$

2) $$\frac{\text{measured g DNA/uL}}{3.65 \times 10^{-15} \text{ g DNA/cell}} = \text{No. cells/uL}$$

EXEMPLE 2

Various amounts of Campylobacter rRNA (100 attomoles (amol)-100 fentomoles (fmol) were suspended in 500 ul of 0.6 M Sodium Chloride, 60 mM sodium citrate, 10 mM Tris, pH 8.0, 50 mM EDTA and 0.05% SDS were hybridized at 56°C for 2.0 hrs. with 0.25 pmol labelled oligonucleotide probe (AE-PM77), the probe being labelled with a chemiluminescent molecule (AE) at its 5' terminus, and 0.5 pmol labelled oligonucleotide probe (AE-PM238), the probe being labelled with a chemiluminescent molecule (AE) at its 5' terminus and 1.0 pmol labelled oligonucleotide probe - (biotin-PM78 labelled at its 5' terminus, the probe being labelled with a first support binding partner (biotin source Aldrich Chemical). The nucleotide sequence of PM77 comprises: 5'-GTA CCG TCA GAA TTC TTC CCT AAG AAA-3'. The hybrid complex was captured with 50 ug of PMP on which a second binding support partner (avidin) had been immobilized. The PMP were separated and washed as described in Example 1. In the detection of the captured hybrid complex, the chemiluminescent reaction reagents were modified as described in Examples 1 and 6 in order to obtain maximum sensitivity. Results were expressed as RLU experimental/RLU background, where RLU background was the chemiluminescent reaction observed in the absence of the added Campylobacter rRNA, and indicate that approximately 100 amol of Campylobacter rRNA was detected, see Figure 2.

EXEMPLE 3

Varying amounts of Campylobacter rRNA (100 amol-100 fmol) were hybridized at 65°C for 1.0 hr. with 1.0 pmol labelled oligonucleotide probe (DNP-PM238), the DNP serving as a first support binding partner, and 0.8 pmol labelled oligonucleotide probe (biotin-PM78), the biotin serving as the second support binding molecule, in 500 ul of buffer (same buffer as described in Example 2). Following hybridization, the hybrids were either captured on the PMP first and then labelled with a labelled chemiluminescent molecules (avidin-AE), forward format, or labelled first with a labelled chemiluminescent material (avidin-AE) and then captured on the PMP, reverse format. In another variation of the format, the labelled oligonucleotide probe (biotin-PM78), the probe being labelled with the second support binding molecule was prelabelled with the labelled chemiluminescent molecules (avidin-AE). This adduct, labelled chemiluminescent molecules and labelled oligonucleotide probe, (avidin-AE/biotin-PM78) was hybridized directly with Campylobacter rRNA along with the probe labelled with the first support binding molecule (DNP-PM238). In the forward format, the hybrid was captured by incubation with 50 ug of labelled solid support (5HI-PMP) for 0.50 hrs. at room temperature (23° C). The PMP were then separated and washed as described in Example 1 and resuspended in 100 ul of buffer containing approximately $5.6 \times 10^5$ RLU of labelled chemiluminescent molecules (avidin-AE). After 2.0 hrs. the PMP were once again separated, washed, resuspended and the chemiluminescent molecules activated. In the reverse format, approximately $5.0 \times 10^7$ RLU of labelled chemiluminescent molecules (avidin-AE) were added to each hybrid solution and incubated for 0.25 hrs. at 65° C. Labelled hybrids were then captured by incubation with 50 ug of labelled PMP (5HI-PMP) for 0.50 hrs. at room temperature (23° C). Samples were processed and the chemiluminescent molecule activated as in the first and second examples. Results of these assays were expressed as signal/background for each amount of rRNA, where background was the signal observed in the absence of rRNA as shown in Figure 3.

EXEMPLE 4

Sandwich hybrids comprised of Campylobacter jejuni rRNA and two derivatized and labelled oligonucleotide probes, one for capture and one for detection, were prepared by combining a various amount ofCampylobacter rRNA in 100 $\mu$l of 60 mM sodium citrate, 10 mM Tris, 0.6 M sodium chloride 50 mM EDTA and 0.05% SDS (hybridization buffer) with 160 fmol of double-labelled probe (5′-AE, 3′-$^{32}$p-PM238) for detection and either 2.0 pmol of an oligonucleotide probe labelled with a first support binding molecule (5′-DNP-PM78) or 2.0 pmol of a probe labelled with a second support binding molecule (5′-biotin-PM78) for capture, and then incubating at 56° C for 2.0 hrs. Following hybridization, 10 ul aliquots were added to 50 ug of capture probe specific solid support (PMP) in 90 ul of 60 mM sodium citratel 16 mM sodium phosphate, 0.72 M sodium chloride, 0.08% BSA, 0.02% Tween-20, and 0.04% sodium azide at pH 7.2 (phosphate assay buffer). Capture specificity was determined by the binding molecule covalently attached to the labelled PMP, 5HI anti-DNP-PMP or avidin-PMP to capture sandwich hybrids containing labelled oligomeric probe (DNP-PM78 or biotin-PM78) respectively. Nonspecific binding of the detector molecules to the solid support (PMP) was assessed in parallel reactions where excess beta-analine-DNP or biotin, respectively, was added to the PMP to block all binding sites prior to the addition of the sandwich hybrid. After incubation for 0.50 hrs. at room temperature (23° C) the bound sandwich hybrid was isolated from excess unhybridized probes and test sample by separation as described in Example 1. The PMP were washed twice with phosphate buffer and resuspended in 100 u of distilled water. Bound sandwich hybrid was detected by chemiluminescent reaction using standard Magic$^R$ Lite reagents and liquid scintillation counting. The results of these assays are summarized in Table 5.

## EXEMPLE 5

The sandwich hybrids described in Example 4 (not bound to PMP) were analyzed by chromotography on a Sepharose CL-6B (Pharmacia) column (Pasteur pipet) which was equilibrated with a buffer containing 0.1 M NaCl, 10.0 mM Tris, 1.0 mM EDTA, pH 7.5. Oligonucleotide probes hybridized to Campylobacter rRNA were shown to elute in the void volume and unhybridized oligonucleotide probes were retained in the column. The column fractions were first analyzed by chemiluminescent reaction and subsequently by liquid scintillation counting. The results of these assays are summarized in Table 6. The data indicate that the extent of hybridization when assessed by either chemiluminescent reaction or liquid scintillation counting are similar. In contrast to Example 4 where the sensitivity for detection of sandwich hybrid captured upon the PMP was significantly better by liquid scintillation counting as opposed to chemiluminescent reaction, when analyzed by chromotography, the sensitivities of the two detection methods were shown to be similar or slightly better for the chemiluminescent reaction.

## EXEMPLE 6

A sandwich hybrid was formed with Campylobacter rRNA by combining 0.5 pmol Campylobacter rRNA, 2.0 pmol labelled oligonucleotide probe (biotin-PM78) and 1.0 pmol labelled oligonucleotide probe (5′-AE-PM238) in 100 ul of hybridization buffer and incubating at 56° C for 2.0 hrs. The resultant hybrid complex anda labelled PMP (avidin-PMP) were mixed, at a ratio of 5.0 fmol of hybrid complex to 250 ug of labelled PMP, and incubated at room temperature (23° C) for 0.50 hrs. to capture the hybrids on the solid support. Bound sandwich hybrid was isolated from excess unhybridized probes and test sample as described in Example 1. The PMP were washed twice with phosphate assay buffer and resuspended in 100 ul of distilled water. Solutions containing the chemiluminescent molecules (AE), the labelled oligomeric DNA probe (AE-PM238) and the sandwich hybrid were suspended in distilled water and divided into 100 ul aliquots. The following parameters associated with the chemiluminescent reaction were examined for purposes of enhancing the detection efficiency of the hybridization assay as described in Examples 4 and 5. First, the time-dependence of the chemiluminescent reaction for the above-described solutions was determined under standard MLA conditions by monitoring the output of the photomultiplier tube of the MLA instrument. In each of the samples the activity peaked within one second of the initiation of the chemiluminescent reaction and then subsided to background level readings within two seconds; thus showing that all of the available light signal was being recorded by the MLA during the standard integration. Since the first step in the chemiluminescent reaction involves attack by hydroperoxide ions on the chemiluminescent molecules (AE) to form an electronically excited molecule, which occurs following addition of reaction reagent 1, the effect

EP 0 425 217 A2

of increasing the incubation period of the immobilized sandwich hybrid in the reaction reagent was determined by varying the delay between injection of the reaction reagents in the MLA sequence from the standard value of 0.1 seconds to 10 min. The results of these assays are summarized in Table 7, showing only negligible increases in the signal for a change in unit time. A matrix of reaction reagents with component concentrations equal to or ten (10) times that of standard reagents were tested for their ability to elicit the chemiluminescent reaction of the AE labelled oligonucleotide probe solutions. All of the modified reaction reagents tested resulted in increased chemiluminescent activity, but the maximum signal enhancement occurred when the acid and base normalities were increased ten-fold (modified reagents) and the hydrogen peroxide and surfactant concentrations were unchanged relative to standard reaction reagents. Under these conditions the chemiluminescent signal detected by the photomultiplier tube from sandwich hybrid in solution increased 2-fold relative to that observed with standard reaction reagents, and an increase of 13-fold was observed for a captured sandwich hybrid. The results of these assays, summarized in Table 8, have revealed conditions that elicit a chemiluminescent response from an AE-oligomer sandwich hybrid bound to a paramagnetic particle comparable to that observed for the same complex in solution.

## EXEMPLE 7

Aliquots of the double-labelled sandwich hybrids formed in Example 4 with biotin-PM78, were captured and processed as previously described except that modified reagents as described in Example 6 were used in the MLA instrument for detection of the chemiluminescent reaction. Following the chemiluminescent reaction, the samples were detected by liquid scintillation counting as described above. The results of these assays are summarized in Table 9; and show that the detection of the chemiluminescent reaction by the modified reagents was enhanced substantially when compared to the standard reagents, while detection by liquid scintillation counting remained relatively unchanged.

## EXEMPLE 8

A sandwich hybrid was formed with Campylobacter rRNA by combining the following: 1.0 pmol Campylobacter rRNA, 1.0 pmol labelled oligonucleotide probe (biotin-PM78) and 2.0 pmol labelled oligonucleotide probe (DNP-PM238) in 100 ul of hybridization buffer and incubating at 650°C for 1.0 hr. Dilutions of this sandwich hybrid were prepared at final concentrations of $2.2 \times 10^{-11}$ M to $2.2 \times 10^{-13}$ M with 60 mM sodium citrate, 10.0 mM Tris, 1.0 mM EDTA, 0.6 M sodium chloride, 0.1% BSA, 0.0 1% sodium azide and 0.02% Tween-20 at pH 7.4 (Tris assay buffer) with SDS at 0.05%. Samples (0.45 ml) of the diluted hybrids, $10^{-14}$ to $10^{-16}$ moles were combined with 50 ug of labelled PMP (5HI-PMP) in 50 ml of Tris assay buffer. Nonspecific binding to the PMP was assessed in parallel reactions where excess beta-alanine-DNP was added to the PMP to block all binding sites prior to the addition of the sandwich hybrid. After incubation for 0.50 hrs. at room temperature (23°C) bound sandwich hybrid was isolated from excess unhybridized oligonucleotide probes and test sample by separation as described above. The PMP were washed once in Tris assay buffer and then the immobilized sandwich hybrids were labelled by resuspending the PMP in 100 $\mu$l of Tris assay buffer containing $6.6 \times 10^6$ RLU of chemiluminescent molecule conjugated second binding partner (avidin-AE) and incubating at room temperature (23° C) for 2.0 hrs. The PMP were washed twice in Tris assay buffer to remove unbound avidin-AE and then resuspended in 100 ul of distilled water. Replicate samples were processed in an MLA instrument with standard and modified reagents. The results of these assays are summarized in Table 10. The combined results of Examples 4 and 5 suggest that solid support bound sandwich containing a chemiluminescent labelled oligonucleotide probe is detected less efficiently than the same hybrid in solution when standard reaction reagents are utilized, i.e. only a fraction of the total hybridized chemiluminescent labelled oligonucleotide captured on the solid support were activated by the standard reaction reagents. In Example 4, the detection by liquid scintillation counting resulted in a significantly improved sensitivity, arbitrarily defined as the hybrid input required to generate a S/B value of 2, relative to the detection by chemiluminescent reaction with standard reaction reagents. However, in Example 5, when the same hybrids were analyzed following chromatographic elution, which achieves a solution phase separation of hybridized and unhybridized probes, sensitivity was comparable by both detection methods. In the first step of the chemiluminescent reaction sequence a quantity of reaction reagent 1 is injected over a short time period (1.2 seconds); the hydrogen peroxide contained in the solution reacts with the chemiluminescent molecules (AE) to form an electronically excited molecule (N-methyzacridone). Then following a brief pause, (0.1 second) an equal quantity of a second

11

reagent is injected over a short time interval (1.2 seconds); as the pH becomes basic the excited molecule undergoes and irreversible reaction involving the emission of light. The emitted light signal is detected by a photomultiplier tube which is in physical proximity to sample, and the MLA instrument microprocessor integrates the photomultiplier tube output for a period of time (2.0 seconds), beginning at the addition of the second reagent. The results of Example 6 show that the chemiluminescent signal decreases to background within two seconds demonstrating that all the available signal has been recorded. This applies to the chemiluminescent reactions of the chemiluminescent molecules alone, chemiluminescent molecules covalently attached to oligonucleotide probes, chemiluminescent oligonucleotide probe labelled sandwich hybrid in solution, and solid support bound chemiluminescent oligonucleotide probe labelled sandwich. Consequently, the low efficiency of the detection of solid phase bound chemiluminescent oligonucleotide probe labelled sandwich hybrid observed with standard reaction reagents was not due to a delayed activation of the chemiluminescent molecules (AE) in the complex. In an attempt to increase the amount of excited molecule (N-methylacridone) generated from the solid support bound chemiluminescent oligonucleotide probe labelled sandwich hybrid in the first reaction reagent during the MLA sequence, thereby increasing the signal generated at the addition of the second reaction reagent, the incubation time of samples in the first reaction reagent was increased by varying the delay between injection of the first and second reaction reagent up to 6000-fold (0.1 seconds to 10 min.). However, only a modest increase in the signal generated was observed, which did not account for the order of magnitude difference in the sensitivity of detection by chemiluminescence versus liquid scintillation counting. Finally, substantial enhancement of the signal from the solid support bound chemiluminescent oligonucleotide probe labelled rRNA sandwich hybrid was obtained when the normality of the reaction reagents were increased tenfold, i.e. modified reagents.

## TABLE 1

| PM # | Location Corresponding to E. Coli 16S rRNA | Base Length | Sequence 5'-3' |
|---|---|---|---|
| 74 | 16S-0163-0214 | 52 | AAC TTT CCC TAC TCA ACT TGT GTT AAG CAG GAG TAT AGA GTA TTA GCA GTC G |
| 75 | 16S-0176-0205 | 30 | TAC TCA ACT TGT GTT AAG CAG GAG TAT AGA |
| 76 | 16S-0163-0204 | 31 | GTT AAG CAG GAG TAT AGA GTA TTA GCA GTC G |
| 77 | 16S-0437-0463 | 27 | GTA CCG TCA GAA TTC TTC CCT AAG AAA |
| 78 | 16S-0641-0671 | 31 | TCT GCC TCT CCC TCA CTC TAG ACT ATG AGT T |
| 79 | 16S-0821-0845 | 25 | ACT AGC ATC CCA ACA ACT AGT GTA C |
| 80 | 16S-0195-0215 | 21 | AAC TTT CCC TAC TCA ACT TGT |
| 122 | 16S-0641-0671 | 31 | TCT GCC TCT CCC TCA CTC TAG ATT ATC AGT T |
| 138 | 16S-0641-0671 | 31 | TCT GCC TCT CCC TCA CTC TAG ATT ATG AGT T |
| 145 | 16S-0156-0185 | 30 | GGA GTA TGG AGT ATT AGC AGT CAT TTC TAA |
| 154 | 16S-0829-0854 | 26 | ACT GCC GTG ACT AGC ACA GCA ACA AC |
| 155 | 16S-1107-1140 | 27 | TGT TAG CAA CTA AAT ACG TGG GTT GCG |
| 238 | 16S-0706-0732 | 27 | GCC TTC GCA ATG GGT ATT CTT GGT GAT |

## TABLE 2

## RNA PURIFICATION PROCEDURE

1. Add 0.1 grams wet weight of cells to a bead beater tube.[a] 2. Discard supernatant. Resuspended cells in 700 ul of buffer by vortexing. 3. Add 50 ul of 20% SDS and 0.1-0.15 mm glass beads to approximately one quarter volume of tube. 4. Add equilibrated phenol to fill tube.[b] 5. Cap and beat for four minutes at room temperature in bead beater. 6. Submerge tubes in water bath (60°C) for fifteen minutes to deproteinate and break up DNA. 7. Dry outside of tube and beat for an additional two minutes. 8. Spin at 3000 RPM (735 x g.) for five minutes in a microfuge. DO NOT USE MICROFUGE AT HIGH SPEEDS! Alternately, spin in the speed vac with no vacuum for ten minutes. 9. Remove nearly all of the aqueous phase (top layer) and the interface to a fresh sterile 1.5 ml microfuge tube (i.e. leave the beads and the phenol behind). 10. Add phenol to fill, and then vortex for 1.5 minutes. 11. Microfuge for 5 minutes at 8,000 RPM (5220 x g.). Remove aqueous phase to new tube taking a small amount of phenol if necessary. 12. Phenol extract (steps 10 and 11) twice more.

TABLE 2

(2 of 3)

13. Phenol/chloroform - (Phenol/chloroform/isoamyl alcohol 25:24:1[c]) extract twice as in steps 10 and 11 except be sure to leave all interface and bottom layer behind after the last extraction. 14. To remaining aqueous phase add 0.1X volume of 3M sodium acetate, pH 5.2. 15. Aliquot in tubes (50 ul per tube). 16. Add two volumes of cold ethanol. Vortex briefly. Precipitate at -70°C overnight. Long term storage of the prep should be in this form and at this temperature. 17. To one of the ethanol precipitate tubes, microfuge at 14,000 RPM (16000 x g.) for ten minutes. Decant ethanol and dr pellets in speed vac (usually five minutes under vacuum is sufficient). 18. To determine optical density resuspend dried pellet in 300 ul of DEPC-treated water. Dilute this sample 1:10 in DEPC wate and measure absorbencies at 260 and 280 nm. Discard the solution that went into the cuvette and store the remainder at -70°C. This is the working solution. a. To prepare cells, grow confluent lawn of bacteria on plates. Each plate generally yields 0.1-0.2 g cells, depending on strain. 1) "Shave" the plates with a sterile razor blade and transfer the cells to a preweighed 50 ml plastic conical centrifuge tube.

TABLE 2

(3 OF 3)

2) Add approximately ten ml LB medium, vortex to resuspend the cells. 3) Spin in Beckman TJ-6R table top centrifuge for 10 minutes at 2400 RPM in TH-4 rotor (1200 x g.). 4) Remove supernatant. 5) Weigh the tube and cells to determine wet cell weight. 6) Add 1 ml LB medium per gram of wet cells. 7) Transfer 0.1 ml wet cells (i.e. 0.1 g) to bead beater tube for RNA preparation. Spin 5 minutes at 3,000 RPM (735 x g.) in microfuge. Discard supernatant. Proceed with this sample as described in main protocol, above. 8) Spin the remainder in table top centrifuge for 10 minutes at 2400 RPM in TH-4 rotor (1200 x g). 9) Remove the liquid. Store cells at -70°C. As an alternative method to harvest the bacteria: 1) Add 1 to 2 ml LB to each plate. Resuspend cells in LB with pipetting action or by dislodging cells with a curved glass rod. 2) Transfer cells to pre-weight 50 ml plastic conical centrifuge tube. Fill to 10 ml with LB medium. 3) Perform steps 3 to 9 as described above. b. To equilibrate phenol, add equal volume of desired buffer, mix vigorously for about 30 seconds, and separate layers by spinning 5 minutes at 500 RPM in TH-4 rotor (50 x g.) in the table-top centrifuge. For twice equilibrated phenol, remove the aqueous phase from the equilibrated phenol and repeat. c. For phenol/chloroform/isoamyl alcohol, mix equal volumes of equilibrated phenol and chloroform/isoamyl alcohol premixed at a ratio of 24:1.

## TABLE 3
### (1 of 5)

| Promega | Organism name | Gel | 74 | 75 | 76 | 77 | 78 | 79 | 80 | 122 | 138 | 145 | 154 | 155 | 238 |
|---|---|---|---|---|---|---|---|---|---|---|---|---|---|---|---|
| PB 101B | Klebsiella pneumoniae | 36,25 | 0 | 0 | 0 | 0 | 0 | | | | | 0 | 0 | 0 | 0 |
| PB 102A | Staphylococcus aureus | 258 | | 0 | | | 0 | 0 | 0 | | | | 0 | 0 | 0 |
| PB 103B | Salmonella enteriditis | 25 | 0 | 0 | 0 | 0 | 0 | 0 | 0 | | | 0 | 0 | 0 | 0 |
| PB 104A1 | Escherichia coli | 1 | | | | | | | | | | | 0 | 0 | |
| PB 104A2 | Escherichia coli | 19 | | 0 | | | 0 | 0 | 0 | | | | 0 | 0 | 0 |
| PB 105B | Proteus mirabilis | 25 | 0 | 0 | 0 | 0 | 0 | 0 | 0 | | | | 0 | 0 | 0 |
| PB 106A1 | Streptococcus Group D | 1 | | | | | | | | | | | 0 | 0 | |
| PB 106A2 | Streptococcus Group D | 19 | | 0 | | | 0 | 0 | 0 | | | | 0 | 0 | 0 |
| PB 107B | Serratia marcescens | 36 | 0 | 0 | 0 | 0 | 0 | 0 | 0 | | | | 0 | 0 | 0 |
| PB 108B | Pseudomonas aeroginosa | 1 | 0 | 0 | 0 | 0 | 0 | 0 | 0 | | | | 0 | 0 | 0 |
| PB 109B | Edwardsiella tarda bg 1 | 1 | 0 | 0 | 0 | 0 | 0 | 0 | 0 | | | | 0 | 0 | 0 |
| PB 110A | Enterobacter agglomerans | 18 | | 0 | | | 0 | 0 | 0 | | 0 | | 0 | 0 | 0 |
| PB 111A | Shigella sonnei | 1 | | 0 | | | 0 | 0 | 0 | | 0 | | 0 | 0 | 0 |
| PB 112B | Yersinia enterocolitica | 36 | 0 | 0 | 0 | 0 | 0 | 0 | 0 | | 0 | | 0 | 0 | 0 |
| PB 113B | Acinetobacter calcoacetic | 36 | 0 | 0 | 0 | 0 | 0 | 0 | 0 | | | | 0 | 0 | 0 |
| PB 114 | Campylobacter jejuni | | | | | | | | | | | | | | |
| PB 115A | Campylobacter jejuni | 459 | | 2 | | | 2 | 2 | 2 | 2 | | | 0 | 2 | 2 |
| PB 116A | Campylobacter jejuni | 1 | 2 | 2 | 2 | 2 | 2 | 2 | 2 | 2 | | 0 | 0 | 2 | 2 |
| PB 117 | Campylobacter jejuni | | | | | | | | | | | | | | |
| PB 118A | Campylobacter coli | 1 | 2 | 2 | 2 | 2 | 2 | 2 | 2 | 2 | | 0 | 0 | 2 | 2 |
| PB 118B | Campylobacter coli | 18 | 2 | 2 | 2 | 2 | 2 | 2 | 2 | 2 | | 0 | 0 | 2 | |
| PB 119A | Campylobacter fetus | 29 | 0 | 0 | 0 | 0 | 2 | 0 | 0 | 2 | 2 | 2 | 2 | 2 | 2 |
| PB 120A | Campylobacter jejuni | 1 | 2 | 2 | 2 | 2 | 2 | 2 | 2 | 2 | | 0 | 0 | 2 | 2 |
| PB 121A | Aeromonas hydrophila | 25 | 0 | 0 | 0 | 0 | 0 | 0 | 0 | | | | 0 | 0 | 0 |
| PB 122A | Aeromonas sorbia | 1 | 0 | 0 | 0 | 0 | 0 | 0 | 0 | | | | 0 | 0 | 0 |
| PB 123A | Citrobacter freundii | 1 | 0 | 0 | 0 | 0 | 0 | 0 | 0 | | | | 0 | 0 | 0 |
| PB 124A | Citrobacter freundii | 1 | 0 | 0 | 0 | 0 | 0 | 0 | 0 | | | | 0 | 0 | 0 |
| PB 125A | Klebsiella pneumoniae | 1 | 0 | 0 | 0 | 0 | 0 | 0 | 0 | | | 0 | 0 | 0 | 0 |
| PB 126A | Enterobacter cloacae | 1 | 0 | 0 | 0 | 0 | 0 | 0 | 0 | | | 0 | 0 | 0 | 0 |
| PB 127A | Pleisiomonas shigelloides | 47,48 | | | | | | | | | | | | | |
| PB 127B | Pleisiomonas shigelloides | 1 | | 0 | | | 0 | 0 | 0 | | 0 | | 0 | 0 | 0 |
| PB 128A | Salmonella arizonae | 36 | 0 | 0 | 0 | 0 | 0 | 0 | 0 | | 0 | | 0 | 0 | 0 |
| PB 129A | Salmonella typhimurium | 49,49 | 0 | 0 | 0 | 0 | 0 | 0 | 0 | | 0 | | 0 | 0 | 0 |
| PB 130A | Shigella sonnei | 1 | 0 | 0 | 0 | 0 | 0 | 0 | 0 | | 0 | | 0 | 0 | 0 |
| PB 131A | Vibrio parahemolyticus | 25 | 0 | 0 | 0 | 0 | 0 | 0 | 0 | | | | 0 | 0 | 0 |
| PB 132A | Streptococcus agalactiae | 25 | 0 | 0 | 0 | 0 | 0 | 0 | 0 | | | | 0 | 0 | 0 |
| PB 133A | Streptococcus faecalis | 47,47 | | | | | | | | | | | | | |
| PB 133B | Streptococcus faecalis | 1 | | 0 | | | 0 | 0 | 0 | | 0 | | 0 | 0 | 0 |
| PB 134A | Streptococcus group C | 47,47 | | | | | | | | | | | | | |
| PB 134B | Streptococcus group C | 18 | | 0 | | | 0 | 0 | 0 | | 0 | | 0 | 0 | 0 |
| PB 135A | Campylobacter jejuni | 28 | 2 | 2 | 2 | 2 | 2 | 2 | 2 | 2 | | | 0 | 2 | 2 |
| PB 136A | Campylobacter fetus | 28 | 0 | 0 | 0 | 0 | 2 | 0 | 0 | 2 | 2 | 2 | 2 | 2 | 2 |
| PB 137A | Campylobacter laridis | 1 | 2 | 2 | 2 | 2 | 2 | 2 | 2 | 2 | | 0 | 0 | 2 | 2 |
| PB 138A | Morganella morganii | 47,47 | | | | | | | | | | | | | |
| PB 138B | Morganella morganii | 1 | | 0 | | | 0 | 0 | 0 | | 0 | | 0 | 0 | 0 |
| PB 139A | Proteus mirabilis | 36,36 | 0 | 0 | 0 | 0 | 0 | 0 | 0 | | | | 0 | 0 | 0 |
| PB 140A | Providencia stuartii | 28 | 0 | 0 | 0 | 0 | 0 | | | | 0 | | 0 | 0 | 0 |
| PB 201A1 | Campylobacter jejuni | 1 | 2 | 2 | 2 | 2 | 2 | 2 | 2 | 2 | | 0 | 0 | 2 | |
| PB 201A2 | Campylobacter jejuni | 1 | 2 | 2 | 2 | 2 | 2 | 2 | 2 | 2 | | 0 | 0 | 2 | 2 |
| PB 202A | Campylobacter jejuni | 1 | 2 | 2 | 2 | 2 | 2 | 2 | 2 | 2 | | 0 | 0 | 2 | 2 |
| PB 203A1 | Campylobacter jejuni | 1 | 2 | 2 | 2 | 2 | 2 | 2 | 2 | 2 | 2 | 0 | 0 | 2 | |
| PB 203A2 | Campylobacter jejuni | 19 | 2 | 2 | 2 | 2 | 2 | 2 | 2 | 2 | 2 | | 0 | 2 | |
| PB 203A3 | Campylobacter jejuni | 19 | 2 | 2 | 2 | 2 | 2 | 2 | 2 | 2 | 2 | | 0 | 2 | 2 |
| PB 203B1 | Campylobacter jejuni | 19 | 2 | 2 | 2 | 2 | 2 | 2 | 2 | 2 | 2 | | 0 | 2 | |
| PB 203B2 | Campylobacter jejuni | 19 | 2 | 2 | 2 | 2 | 2 | 2 | 2 | 2 | 2 | | 0 | 2 | |
| PB 203C | Campylobacter jejuni | 19 | 2 | 2 | 2 | 2 | 2 | 2 | 2 | 2 | 2 | | 0 | 2 | |
| PB 204A | Campylobacter jejuni | 1 | 2 | 2 | 2 | 2 | 2 | 2 | 2 | | | 0 | 0 | 2 | 2 |
| PB 205A | Campylobacter jejuni | 25 | | 2 | | | 2 | 2 | 2 | 2 | | | 0 | 1 | 2 |
| PB 206A | Campylobacter coli | 28 | 2 | 2 | 2 | 2 | 2 | 2 | 2 | 2 | | 0 | 0 | 2 | 2 |
| PB 207A | Campylobacter jejuni | 28 | 2 | 2 | 2 | 2 | 2 | 2 | 2 | 2 | | 0 | 0 | 2 | 2 |

## TABLE 3

### (2 of 5)

| Promega # | Organism name | Gel | 74 | 75 | 76 | 77 | 78 | 79 | 80 | 122 | 138 | 145 | 154 | 155 | 238 |
|---|---|---|---|---|---|---|---|---|---|---|---|---|---|---|---|
| PB 208A | Campylobacter jejuni | 28 | 2 | 2 | 2 | 2 | 2 | 2 | 2 | | 2 | 0 | 2 | | 2 |
| PB 209A | Campylobacter jejuni | 1 | 2 | 2 | 2 | 2 | 2 | 2 | 2 | | | 0 | 2 | | 2 |
| PB 210A | Campylobacter jejuni | 79 | | | 2 | | 2 | 2 | 2 | | 2 | 0 | 2 | | 2 |
| PB 211A | Campylobacter jejuni | 1 | 2 | 2 | 2 | 2 | 2 | 2 | 2 | 2 | 2 | 0 | 2 | | 2 |
| PB 212A | Campylobacter jejuni | 1 | 2 | 2 | 2 | 2 | 2 | 2 | 2 | | 2 | 0 | 2 | | 2 |
| PB 213A | Campylobacter jejuni | 47 | | | | | | | | | | | | | |
| PB 213B | Campylobacter jejuni | 37 | | | | | | | | | | | | | |
| PB 213C | Campylobacter jejuni | 1 | | | 2 | | 2 | 2 | 2 | | | 0 | 2 | | 2 |
| PB 214A | Campylobacter jejuni | 1 | 2 | 2 | 2 | 2 | 2 | 2 | 2 | | 2 | 0 | 2 | | 2 |
| PB 214B | Campylobacter jejuni | 19 | 2 | 2 | 2 | 2 | 2 | 2 | 2 | | 2 | 0 | 2 | | |
| PB 215A | Campylobacter coli | 48 | 2 | 2 | 2 | 2 | 2 | 2 | 2 | | 2 | 0 | 2 | | 2 |
| PB 216A | Campylobacter jejuni | 49 | | | 2 | | 2 | 2 | 2 | | 2 | 0 | 2 | | 2 |
| PB 217A | Campylobacter jejuni | 258 | | | 2 | | 2 | 2 | 2 | | 2 | 0 | 2 | | 2 |
| PB 218A | Campylobacter jejuni | 18 | 2 | 2 | 2 | 2 | 2 | 2 | 2 | | 2 | 0 | 2 | | 2 |
| PB 219A | Campylobacter jejuni | 28 | 2 | 2 | 2 | 2 | 2 | 2 | 2 | | 2 | 0 | 2 | | 2 |
| PB 220A | Campylobacter jejuni | 48 | | | 2 | | 2 | 2 | 2 | | 2 | 0 | 2 | | 2 |
| PB 221A | Campylobacter coli | 18 | 2 | 2 | 2 | 2 | 2 | 2 | 2 | | 2 | 0 | 2 | | 2 |
| PB 222A | Campylobacter jejuni | 48 | 2 | 2 | 2 | 2 | 2 | 2 | 2 | | 2 | 0 | 2 | | 2 |
| PB 223A | Campylobacter jejuni | 18 | 2 | 2 | 2 | 2 | 2 | 2 | 2 | | 2 | 0 | 2 | | 2 |
| PB 224A | Campylobacter jejuni | 18 | 2 | 2 | 2 | 2 | 2 | 2 | 2 | | 2 | 0 | 2 | | 2 |
| PB 225A | Campylobacter coli | 49 | 2 | 2 | 2 | 2 | 2 | 2 | 2 | | 2 | 0 | 2 | | 2 |
| PB 226A | Campylobacter coli | 1 | 2 | 2 | 2 | 2 | 2 | 2 | 2 | | 2 | 0 | 0 | 2 | 2 |
| PB 227A | Campylobacter coli | 49 | 2 | 2 | 2 | 2 | 2 | 2 | 2 | | 2 | | 1 | | 2 |
| PB 228A | Campylobacter jejuni | | | | | | | | | | | | | | |
| PB 228B | Campylobacter jejuni | 48 | | | 2 | | 2 | 2 | 2 | | 2 | 0 | 2 | | 1 |
| PB 229A | Campylobacter coli | 47 | | | | | | | | | | | | | |
| PB 229B | Campylobacter coli | 458 | | | 2 | | 2 | 2 | 2 | | 2 | 0 | 2 | | 2 |
| PB 230A | Campylobacter jejuni | 47 | | | | | | | | | | | | | |
| PB 230B | Campylobacter jejuni | 458 | | | 2 | | 2 | 2 | 2 | | 2 | 0 | 2 | | 2 |
| PB 231A | Campylobacter coli | 28 | 2 | 2 | 2 | 2 | 2 | 2 | 2 | | 2 | 0 | 2 | | 2 |
| PB 232A | Campylobacter jejuni | 47 | | | | | | | | | | | | | |
| PB 232B | Campylobacter jejuni | 458 | | | 2 | | 2 | 2 | 2 | | 2 | 0 | 2 | | 2 |
| PB 233A | Campylobacter jejuni | 458 | | | 2 | | 2 | 2 | 2 | | 2 | 0 | 2 | | 2 |
| PB 234A | Campylobacter jejuni | 458 | | | 2 | | 2 | 2 | 2 | | 2 | 0 | 2 | | 2 |
| PB 235A | Campylobacter coli | 358 | | | 2 | | 2 | 2 | 2 | | 2 | 0 | 2 | | 2 |
| PB 236A | Campylobacter jejuni | 37 | | | | | | | | | | | | | |
| PB 236B | Campylobacter jejuni | 1 | | | 2 | | 2 | 2 | 2 | | 2 | 0 | 2 | | 2 |
| PB 237A | Campylobacter jejuni | 49 | | | 2 | | 2 | 2 | 2 | | 1 | 0 | 2 | | 2 |
| PB 238A | Campylobacter jejuni | 46 | | | | | | | | | | | | | |
| PB 238B | Campylobacter jejuni | 1 | | | 2 | | 2 | 2 | 2 | | 2 | 0 | 2 | | 2 |
| PB 239A | Campylobacter jejuni | 49 | | | 2 | | 2 | 2 | 2 | | 2 | 0 | 2 | | 2 |
| PB 240A | Campylobacter coli | 49 | | | 2 | | 2 | 2 | 2 | | 2 | 0 | 2 | | 2 |
| PB 301A | Campylobacter jejuni | 36 | | | 2 | | 2 | 2 | 2 | | 2 | 0 | 2 | | 2 |
| PB 302A | Campylobacter jejuni | 468 | | | 2 | | 2 | 2 | 2 | | 2 | 0 | 2 | | 2 |
| PB 303A | Campylobacter jejuni | 468 | | | 1 | | 2 | 2 | 2 | | 2 | 0 | 2 | | 2 |
| PB 304A | Campylobacter jejuni | 46 | | | 1 | | 2 | 1 | 2 | | 1 | 0 | 2 | | 2 |
| PB 305A | Campylobacter jejuni | 37 | | | | | | | | | | | | | |
| PB 305B | Campylobacter jejuni | 1 | | | 2 | | 2 | 2 | 2 | | 2 | 0 | 2 | | 2 |
| PB 306A | Campylobacter jejuni | 469 | | | 2 | | 2 | 2 | 2 | | 2 | 0 | 2 | | 2 |
| PB 307A | Campylobacter coli | 18 | | | 2 | | 2 | 2 | 2 | | 2 | 0 | 2 | | 2 |
| PB 308A | Campylobacter coli | 18 | | | 2 | | 2 | 2 | 2 | | 2 | 0 | 2 | | 2 |
| PB 309A | Campylobacter coli | 1 | | | 2 | | 2 | 2 | 2 | | 2 | 0 | 2 | | 2 |
| PB 310A | Campylobacter coli | 18 | | | 2 | | 2 | 2 | 2 | | 2 | 0 | 2 | | 2 |
| PB 311A | Campylobacter jejuni | 47 | | | | | | | | | | | | | |
| PB 311B | Campylobacter jejuni | 48 | | | 2 | | 2 | 2 | 2 | | 2 | 0 | 2 | | 2 |
| PB 312A | Campylobacter coli | 46 | | | | | | | | | | | | | |
| PB 312B | Campylobacter coli | 48 | | | 2 | | 2 | 2 | 2 | | 2 | 0 | 2 | | 2 |
| PB 313A | Campylobacter jejuni | 47 | | | | | | | | | | | | | |
| PB 313B | Campylobacter jejuni | 48 | | | 2 | | 2 | 2 | 2 | | 2 | 0 | 2 | | 2 |
| PB 314A | Campylobacter jejuni | 47 | | | | | | | | | | | | | |

TABLE 3

| Promega # | Organism name | Gel | 74 | 75 | 76 | 77 | 78 | 79 | 80 | 122 | 138 | 145 | 154 | 155 | 238 |
|---|---|---|---|---|---|---|---|---|---|---|---|---|---|---|---|
| PB 314B | Campylobacter jejuni | 48 | | 2 | | 2 | 2 | 2 | | | 2 | | 0 | 2 | 2 |
| PB 315A | Campylobacter coli | 49 | | 2 | | 2 | 2 | 2 | | | 2 | | 0 | 2 | 2 |
| PB 316A | Campylobacter jejuni | 46 | | | | | | | | | | | | | |
| PB 316B | Campylobacter jejuni | 48 | | 2 | | 2 | 2 | | | | 2 | | 0 | 2 | 2 |
| PB 317A | Campylobacter coli | 38 | | 2 | | 2 | 2 | 2 | | | 2 | | 0 | 2 | 2 |
| PB 318A | Campylobacter jejuni | 47 | | | | | | | | | | | | | |
| PB 318B | Campylobacter jejuni | 48 | | 2 | | 2 | 2 | 2 | | | 2 | | 0 | 2 | 2 |
| PB 319A | Campylobacter jejuni | 47 | | | | | | | | | | | | | |
| PB 319B | Campylobacter jejuni | 48 | | 2 | | 2 | 2 | 2 | | | 2 | | 0 | 2 | 2 |
| PB 320A | Campylobacter coli | 47 | | | | | | | | | | | | | |
| PB 320B | Campylobacter coli | 18 | | 2 | | 2 | 2 | 2 | | | 2 | | 0 | 2 | 2 |
| PB 321A | Campylobacter jejuni | 47 | | | | | | | | | | | | | |
| PB 321B | Campylobacter jejuni | 48 | | 2 | | 2 | 2 | 2 | | | 2 | | 0 | 2 | 2 |
| PB 322A | Yersinia enterocolitica | 49 | 0 | 0 | 0 | 0 | 0 | | | | 0 | | 0 | 0 | 0 |
| PB 323A | Enterobacter aerogenes | 25 | 0 | 0 | 0 | 0 | 0 | 0 | 0 | | | | 0 | 0 | 0 |
| PB 324A | Enterobacter cloacae | 1,1 | 0 | 0 | 0 | 0 | 0 | 0 | 0 | | | | 0 | 0 | 0 |
| PB 325A | Morganella morganii | 1 | 0 | 0 | 0 | 0 | 0 | 0 | 0 | | | | 0 | 0 | 0 |
| PB 326A | Providencia rettgeri | 1 | 0 | 0 | 0 | 0 | 0 | 0 | 0 | | | | 0 | 0 | 0 |
| PB 327A | Acinetobacter calcoacetic | 48 | 0 | 0 | 0 | 0 | 0 | | | | 0 | | 0 | 0 | 0 |
| PB 328A | Escherichia coli A-D | 47 | | | | | | | | | | | | | |
| PB 329A | Salmonella st paul | | | 0 | | 0 | 0 | 0 | | | | | 0 | 0 | 0 |
| PB 330A | Salmonella give | 28 | 0 | 0 | 0 | 0 | 0 | 0 | 0 | | 0 | | 0 | 0 | 0 |
| PB 331A | Salmonella mississippi | 28 | 0 | 0 | 0 | 0 | 0 | 0 | 0 | | 0 | | 0 | 0 | 0 |
| PB 332A | Shigella boydii | 1 | 0 | 0 | 0 | 0 | 0 | 0 | 0 | 0 | 0 | | 0 | 0 | 0 |
| PB 333A | Shigella dysenteriae | 25 | 0 | 0 | 0 | 0 | 0 | 0 | 0 | 0 | 0 | | 0 | 0 | 0 |
| PB 334A | Shigella flexneri | 1 | 0 | 0 | 0 | 0 | 0 | 0 | 0 | 0 | 0 | | 0 | 0 | 0 |
| PB 335A | Streptococcus agalactiae | 47 | | | | | | | | | | | | | |
| PB 335B | Streptococcus agalactiae | 1 | | 0 | | 0 | 0 | 0 | | | 0 | | 0 | 0 | 0 |
| PB 336A | Candida albicans | 25 | 0 | 0 | 0 | 0 | 0 | 0 | 0 | | | | 0 | 0 | 0 |
| PB 337A | Candida tropicalis | 36 | 0 | 0 | 0 | 0 | 0 | 0 | 0 | | | | 0 | 0 | 0 |
| PB 338A | Staphylococcus aureus | 258 | | 0 | | 0 | 0 | 0 | | | | | 0 | 0 | 0 |
| PB 339A | Staphylococcus epidermidi | 19 | | 0 | | | 0 | 0 | | | | | | | |
| PB 340A | Providencia stuartii | 28 | 0 | 0 | 0 | 0 | 0 | 0 | 0 | | | | 0 | 0 | 0 |
| PB 401A | Salmonella schwarzengrund | 18 | 0 | 0 | 0 | 0 | 0 | 0 | 0 | | 0 | 0 | 0 | 0 | 0 |
| PB 402A | Salmonella typhi | | | 0 | | 0 | 0 | 0 | | | | | 0 | 0 | 0 |
| PB 403A1 | Wolinella species | 1 | 0 | 0 | 0 | 0 | 1 | 0 | | 1 | 0 | | 0 | 0 | |
| PB 403A2 | Wolinella species | 36 | 0 | 0 | 0 | 0 | 1 | 0 | | 1 | 0 | | 0 | 0 | 0 |
| PB 403A3 | Wolinella species | 25 | 0 | 0 | 0 | 0 | 1 | 0 | | 1 | 0 | | 0 | 0 | |
| PB 404A | Wolinella curva | 479 | | | | | | | | | | | | | |
| PB 404B1 | Wolinella curva | 48 | | 0 | | 0 | 1 | 0 | | 1 | | | 0 | 0 | |
| PB 404B2 | Wolinella curva | 48 | | 0 | | 0 | 1 | 0 | | 1 | 1 | | 0 | 0 | |
| PB 404B3 | Wolinella curva | 36 | | 0 | | 0 | 1 | 0 | | 1 | | | 0 | 0 | 2 |
| PB 405A | Wolinella recta | 48 | 2 | 0 | 2 | 0 | 2 | 0 | | 2 | 2 | 0 | 0 | 0 | 0 |
| PB 406A1 | Wolinella succinogenes | 1 | 0 | 0 | 0 | 0 | 0 | 0 | | | 0 | | 0 | 0 | |
| PB 406A2 | Wolinella succinogenes | 25 | 0 | 0 | 0 | 0 | 0 | 0 | | | 0 | 0 | 0 | 0 | 0 |
| PB 406A3 | Wolinella succinogenes | 25 | 0 | 0 | 0 | 0 | 0 | 0 | | | 0 | | 0 | 0 | |
| PB 407A | Campylobacter hyointestinl | | 1 | 0 | 0 | 0 | 2 | 0 | 0 | 2 | 2 | | | | 2 |
| PB 408A | Campylobacter cinaedi | 47 | | | | | | | | | | | | | |
| PB 408B | Campylobacter cinaedi | 479 | | 0 | | 0 | 0 | 0 | | | 0 | | 0 | 0 | 1 |
| PB 408C | Campylobacter cinaedi | 479 | | 0 | | 0 | 0 | 0 | | | 0 | | 0 | 0 | |
| PB 408D | Campylobacter cinaedi | 479 | | 0 | | 0 | 0 | 0 | | | 0 | | 0 | 0 | |
| PB 409A | Escherichia coli O157:H7 | 1 | 0 | 0 | 0 | 0 | 0 | | | | 0 | | 0 | 0 | 0 |
| PB 410A | Escherichia coli O157:H7 | 1 | 0 | 0 | 0 | 0 | 0 | | | | 0 | | 0 | 0 | 0 |
| PB 411A1 | Vibrio cholerae | 1,1 | 0 | 0 | 0 | 0 | 0 | | | 0 | 0 | 0 | 0 | | |
| PB 411A2 | Vibrio cholerae | 1 | 0 | 0 | 0 | 0 | 0 | | | 0 | 0 | 0 | 0 | 0 | 0 |
| PB 412A | Vibrio cholerae | 47,1 | | 0 | | 0 | 0 | 0 | | | 0 | | 0 | 0 | 0 |
| PB 413A | Bact. fragilis | 1 | 0 | 0 | 0 | 0 | 0 | | | | 0 | 0 | 0 | 0 | 0 |
| PB 414A1 | Bact. fragilis | 1 | 0 | 0 | 0 | 0 | 0 | | | 0 | 0 | | 0 | 0 | 0 |
| PB 414A2 | Bact. fragilis | 25 | 0 | 0 | 0 | 0 | 0 | | | 0 | 0 | | 0 | 0 | |

| Promega | Organism name | Gel | 74 | 75 | 76 | 77 | 78 | 79 | 80 | 122 | 138 | 145 | 154 | 155 | 238 |
|---|---|---|---|---|---|---|---|---|---|---|---|---|---|---|---|
| PB 415A | Bact. thetaiotamicron | 1 | 0 | 0 | 0 | 0 | 0 | | | | 0 | 0 | 0 | 0 | 0 |
| PB 416A | Bact. vulgatus | 1 | 0 | 0 | 0 | 0 | 0 | | | 0 | 0 | 0 | 0 | 0 | 0 |
| PB 417A | Clostridium perfringens | | 0 | 0 | 0 | 0 | 0 | | | | | | | | |
| PB 417B | Clostridium perfringens | 1 | | 0 | | | 0 | 0 | 0 | | 0 | 0 | 0 | 0 | 0 |
| PB 418A | Clostridium perfringens | 59 | | 0 | | | 0 | 0 | 0 | | 0 | | 0 | 0 | 0 |
| PB 419A1 | Clostridium ramosum | 1 | 0 | 0 | 0 | 0 | 0 | | | 0 | 0 | | 0 | 0 | |
| PB 419A2 | Clostridium ramosum | 1 | 0 | 0 | 0 | 0 | 0 | | | 0 | 0 | | 0 | 0 | 0 |
| PB 420A1 | Clostridium sordellii | 18 | 0 | 0 | 0 | 0 | 0 | | | | 0 | | 0 | 0 | |
| PB 420A2 | Clostridium sordellii | 18 | 0 | 0 | 0 | 0 | 0 | | | | 0 | | 0 | 0 | |
| PB 420A3 | Clostridium sordellii | 18 | 0 | 0 | 0 | 0 | 0 | | | | 0 | | 0 | 0 | 0 |
| PB 421A1 | Clostridium septicum | 1 | 0 | 0 | 0 | 0 | 0 | | | | 0 | | 0 | 0 | |
| PB 421A2 | Clostridium septicum | 36 | 0 | 0 | 0 | 0 | 0 | | | | 0 | | 0 | 0 | 0 |
| PB 422A | Clostridium tetani | 47 | | | | | | | | | | | | | |
| PB 423A | Clostridium tetani | 1 | 0 | 0 | 0 | 0 | 0 | | | | 0 | | 0 | 0 | 0 |
| PB 424A | Peptostrep. anaerobius | 1 | 0 | 0 | 0 | 0 | 0 | | | | 0 | | 0 | 0 | 0 |
| PB 425A | Bact. distasonis | 36 | | 0 | | | 0 | 0 | 0 | | 0 | 0 | 0 | 0 | 0 |
| PB 426A | Bact. multiacidus | 1 | 0 | 0 | 0 | 0 | 0 | | | | 0 | 0 | 0 | 0 | 0 |
| PB 427A | Bifidobacterium breve | | | | | | | | | | | | | | |
| PB 428A | Fusobacterium nucleotum | | | | | | | | | | | | | | |
| PB 428B | Fusobacterium nucleotum | 26 | | 0 | | | 0 | 0 | 0 | | | | 0 | 0 | 0 |
| PB 429A | Bact. melaninogenicus | 36 | 0 | 0 | 0 | 0 | 0 | | | | 0 | 0 | 0 | 0 | 0 |
| PB 430A | Campylobacter upsaliensis | 1 | 1 | 0 | 0 | 1 | 2 | 0 | 0 | | 2 | 0 | 0 | 0 | 2 |
| PB 431A | Campylobacter upsaliensis | 36 | 0 | 0 | 0 | 0 | 1 | 0 | 0 | 1 | 1 | | 0 | 0 | 2 |
| PB 432A | Anaerobiospirillum soc. | 1 | | 0 | | | 0 | 0 | 0 | | 0 | | 0 | 0 | 0 |
| PB 433A | Campylobacter fennelliae | 479,479 | | 0 | | | 0 | 0 | 0 | | 0 | | 0 | 0 | 1 |
| PB 433B | Campylobacter fennelliae | 479 | | 0 | | | 0 | 0 | 0 | | 0 | | 0 | 0 | |
| PB 433C | Campylobacter fennelliae | 479 | | 0 | | | 0 | 0 | 0 | | 0 | | 0 | 0 | |
| PB 434A | Campylobacter fennelliae | 479,479 | | 0 | | | 0 | 0 | 0 | | 0 | | 0 | 0 | 0 |
| PB 434B | Campylobacter fennelliae | 479 | | 0 | | | 0 | 0 | 0 | | 0 | | 0 | 0 | |
| PB 434C | Campylobacter fennelliae | 479 | | 0 | | | 0 | 0 | 0 | | 0 | | 0 | 0 | |
| PB 435A | Eubacterium aerofaciens | 36 | | 0 | | | 0 | 0 | 0 | | 0 | 0 | 0 | 0 | 0 |
| PB 436A | Peptostreptococcus produc | 36 | | 0 | | | 0 | 0 | 0 | | 0 | 0 | 0 | 0 | 0 |
| PB 439A | Coprococcus eutactus | 1 | | | | | | | | | | | | | |
| PB 439B1 | Coprococcus eutactus | 1 | | 0 | | | 0 | 0 | 0 | | | 0 | 0 | 0 | 0 |
| PB 439B2 | Coprococcus eutactus | 1 | | 0 | | | 0 | 0 | 0 | | | 0 | 0 | 0 | |
| PB 440A | Campylobacter concisus | 1 | | 0 | | | 0 | 2 | 0 | | 2 | | 0 | 0 | 2 |
| PB 501A | Bact. gracilis | 46 | | 0 | | | 0 | 0 | 0 | | 0 | 1 | 1 | 0 | 0 |
| PB 501B | Bact. gracilis | 48 | | 0 | | | 0 | 0 | 0 | | 0 | 1 | 1 | 0 | |
| PB 502A | Bact. ureolyticus | 468 | | 0 | | | 0 | 0 | 0 | | 0 | 0 | 0 | 1 | 0 |
| PB 503A | Veillonella parvula | 1 | | 0 | | | 0 | 0 | 0 | | 0 | | 0 | 0 | 0 |
| PB 504A | Streptococcus mitis | 1 | | 0 | | | 0 | 0 | 0 | | 0 | | 0 | 0 | 0 |
| PB 505A | Alcaligenes faecalis | 37 | | | | | 0 | 0 | 0 | | | | | | |
| PB 505B | Alcaligenes faecalis | 1 | | 0 | | | 0 | 0 | 0 | | | | 0 | 0 | 0 |
| PB 506A | Bacillus cereus | 1 | | 0 | | | 0 | 0 | 0 | | 0 | | 0 | 0 | 0 |
| PB 507A | Streptococcus pyogenes | 18 | | 0 | | | 0 | 0 | 0 | | 0 | | 0 | 0 | 0 |
| PB 508A | Neisseria gonorrhoeae | 1 | | 0 | | | 0 | 0 | 0 | | 0 | 0 | 0 | 0 | 0 |
| PB 509A | Campylobacter fetus | 38 | | 0 | | | 0 | 2 | 0 | | 2 | 2 | 2 | 2 | 2 |
| PB 510A | Campylobacter coli | 1 | | 2 | | | 1 | 2 | 0 | | 2 | 0 | 0 | 2 | 2 |
| PB 510B | Campylobacter coli | 19 | | 2 | | | 1 | 2 | 0 | | 2 | 0 | 0 | 2 | |
| PB 511A | Campylobacter fetus | 38 | | 0 | | | 0 | 2 | 0 | | 2 | 2 | 2 | 2 | 2 |
| PB 512A | Campylobacter fetus | 48 | | 0 | | | 0 | 2 | 0 | | 2 | 2 | 2 | 2 | 2 |
| PB 513A | Campylobacter fetus | 38 | | 0 | | | 0 | 2 | 0 | | | | 2 | 2 | 2 |
| PB 514A1 | Campylobacter fetus | 38 | | 0 | | | 0 | 2 | 0 | | 2 | | 2 | 2 | |
| PB 514A2 | Campylobacter fetus | 18 | | 0 | | | 0 | 2 | 0 | | 2 | | 2 | 2 | 2 |
| PB 515A | Campylobacter fetus | 38 | | 0 | | | 0 | 2 | 0 | | | | 2 | 2 | 2 |
| PB 516A | Campylobacter fetus | 38 | | 0 | | | 0 | 2 | 0 | | 2 | 2 | 2 | 2 | 2 |
| PB 517A | Campylobacter fetus | 38 | | 0 | | | 0 | 2 | 0 | | 2 | 2 | 2 | 2 | 2 |
| PB 518A | Campylobacter laridis | 1 | | 2 | | | 2 | 2 | 2 | | 2 | 0 | 0 | 2 | 2 |
| PB 519A | Campylobacter laridis | 1 | | 2 | | | 2 | 2 | 2 | | 2 | 0 | 0 | 2 | 2 |
| PB 519B | Campylobacter laridis | 1 | | 2 | | | 2 | 2 | 2 | | 2 | 0 | 0 | 2 | |

## Table 3

## (5 of 5)

| Promega | Organism name | Gel | 74 | 75 | 76 | 77 | 78 | 79 | 80 | 122 | 138 | 145 | 154 | 155 | 238 |
|---|---|---|---|---|---|---|---|---|---|---|---|---|---|---|---|
| PB 520A | Campylobacter laridis | 1 | | 1 | | 2 | | 2 | | 2 | | 2 | 0 | 2 | 2 |
| PB 521A | Campylobacter laridis | 47 | | | | | | | | | | | | | |
| PB 521B | Campylobacter laridis | 1 | | 2 | | 2 | | 2 | | 2 | | | 0 | 2 | 2 |
| PB 522A1 | Campylobacter pylori | 19 | | 0 | | 0 | | 0 | | 0 | 0 | | 0 | 0 | |
| PB 522A2 | Campylobacter pylori | 18 | | 0 | | 0 | | 0 | | 0 | 0 | | 0 | 0 | 0 |
| PB 523A | Campylobacter pylori | 19 | | 0 | | 0 | | 0 | | 0 | 0 | 0 | 0 | 0 | 0 |
| PB 524A | Campylobacter pylori | 58 | | 0 | | 0 | | 1 | | | | | 0 | 0 | 1 |
| PB 525A | Campylobacter pylori | 18 | | 0 | | 0 | | 2 | | | | | 0 | 0 | 1 |
| PB 533A1 | Escherichia coli | 1 | 0 | 0 | 0 | 0 | 0 | 0 | 0 | 0 | 0 | 0 | 0 | 0 | |
| PB 533A2 | Escherichia coli | 1 | 0 | 0 | 0 | 0 | 0 | 0 | 0 | 0 | 0 | 0 | 0 | 0 | 0 |
| PB 533B | Escherichia coli | 18 | 0 | 0 | 0 | 0 | 0 | 0 | 0 | 0 | 0 | 0 | 0 | 0 | 0 |
| PB 603A | Lactobacillus acidophilus | 1 | | 0 | | 0 | | 0 | | 0 | | | 0 | 0 | 0 |
| PB 604A | Fusobacterium necrophorum | 45 | | 0 | | 0 | | 0 | | 0 | | | 0 | 0 | 0 |
| PB 701A | Clostridium difficile | 258 | | 0 | | 0 | | 0 | | 0 | | | 0 | 0 | 0 |

The first column indicates each strain number used at Promega Corporation. The second column contains the genus and species name for each organism. The third column indicates the interpretation after agarose gel electrophoresis of the integrity of the rRNA purified from each strain. Two major bands were expected for each sample, the top band was presumed to be 23S rRNA, and the lower band was presumed to be 16S rRNA. Extra bands were sometimes observed and sometimes bands were missing according to the following key: 1 = both bands were present and normal intensity 2 = the top band was underrepresented 3 = the top band was strongly underrepresented 4 = the top band was missing 5 = the bottom band was underrepresented 6 = the bottom band was strongly underrepresented 7 = the bottom band was missing 8 = preparation has one extra band 9 = preparation has two or more extra bands When the column labelled "Gel" has multiple numbers in it, all listed cases apply. In the rest of the table, (0) indicates no hybridization, (2) indicates strong hybridization, and (1) indicates weak hybridization. Blank spaces indicate no testing or interpretation of a particular probe with a particular strain.

## TABLE 4

### CAMPYLOBACTER PROBES – – HYBRIDIZATION SUMMARY

|  | 74 | 75 | 76 | 77 | 78 | 79 | 80 | 122 | 138 | 145 | 154 | 155 | 238 |
|---|---|---|---|---|---|---|---|---|---|---|---|---|---|
| C. jejuni, coli, laridis | + | + | + | + | + | + | + | + | + | – | – | + | + |
| C. fetus | – | – | – | – | + | – | – | + | + | + | + | + | + |
|  |  |  |  |  |  |  |  |  |  |  |  |  |  |
| C. cinaedi | 0 | – | 0 | – | – | – | 0 | 0 | – | 0 | – | – | + |
| C. hyointestinalis | + | – | – | – | + | – | – | + | + | 0 | 0 | 0 | + |
| C. pylori | 0 | – | 0 | – | – | – | 0 | 0 | – | – | – | – | +/– |
| C. upsaliensis | – | – | – | + | + | – | – | + | + | – | – | – | + |
|  |  |  |  |  |  |  |  |  |  |  |  |  |  |
| Bacteroides gracilis | 0 | – | 0 | – | – | – | 0 | 0 | – | + | – | – | – |
| Bacteroides ureolyticus | 0 | – | 0 | – | – | 0 | 0 | – | – | – | – | + | – |
| Wolinella curva | 0 | – | 0 | – | + | – | 0 | 0 | + | + | – | – | + |
| Wolinella recta | + | – | + | – | + | 0 | 0 | + | + | – | – | – | – |
| All others | – | – | – | – | – | – | – | – | – | – | – | – | – |

0 = NOT DETERMINED
+ = Hybridization
– = No Hybridization

## TABLE 5

| (Fmol) rRNA Input | 5H1 - PMP Capture | | | | Streptavidin - PMP Capture | | | |
|---|---|---|---|---|---|---|---|---|
| | CHEMILUMINESENCE | | LIQUID SCINTILLATION | | CHEMILUMINESENCE | | LIQUID SCINTILATION | |
| | (RLU) Signal | S/B [1] | (CPM) Signal | S/B [1] | (RLU) Signal | S/B [2] | (CPU) Signal | S/B [2] |
| 0.1 | 645 | 1.0 | 66 | 1.9 | 470 | 1.1 | 66 | 1.9 |
| 1 | 1,595 | 2.5 | 438 | 13 | 885 | 2.0 | 454 | 13 |
| 10 | 8,970 | 14 | 3,313 | 97 | 4,200 | 9.4 | 3,487 | 100 |
| 100 | 14,500 | 22 | 4,947 | 145 | 10,050 | 23 | 5,909 | 169 |
| NSB: | | | | | | | | |
| 0.1 | 665 | | 38 | | 430 | | 31 | |
| 1 | 635 | $\bar{x} = 645$ | 29 | $\bar{x} = 34$ | 460 | $\bar{x} = 445$ | 33 | $\bar{x} = 35$ |
| 10 | 625 | | 38 | | 450 | | 38 | |
| 100 | 660 | | 31 | | 440 | | 38 | |
| Sensitivity [3] | 0.71 Fmols | | 0.10 Fmols | | 1.0 Fmols | | 0.11 Fmols | |

1: Signal to Background (S/B) is the dividend of the chemiluminescent signal observed (Relative Light Units (RLU)) at a given rRNA input divided by the mean of the non-specifically bound (NSB) chemiluminescent signal observed at all rRNA inputs.

2: Signal to Background (S/B) is the dividend of the liquid scintillation signal observed (counts of radioactivity) (CPM) at a given rRNA input divided by the mean of the non-specifically bound (NSB) liquid scintillation signal observed at all rRNA inputs.

3: Sensitivity is defined as the amount of sandwich hybrid, in femtomoles ($1 \times 10^{1-15}$) required to generate S/B of 2; calculated by linear interpolation.

TABLE 6

| (fmol)<br>rRNA Input | Capture Probe | Hybrid RLU | % Hybridization | Hybrid CPM | % Hybridization |
|---|---|---|---|---|---|
| 0.1 | DNP-PM 78 | 1,200 | 46 | - | - |
| 1 | DNP-PM 78 | 18,000 | 77 | 750 | 97 |
| 10 | DNP-PM 78 | 140,000 | 53 | 4,530 | 69 |
| 100 | DNP-PM 78 | 334,000 | 56 | 8,360 | 65 |
| 0.1 | Biotin - PM 78 | 2,000 | 65 | - | - |
| 1 | Biotin - PM 78 | 22,600 | 76 | 280 | 51 |
| 10 | Biotin - PM 78 | 189,000 | 64 | 5,260 | 80 |
| 100 | Biotin - PM 78 | 336,000 | 66 | 8,900 | 69 |

EP 0 425 217 A2

**TABLE 7**

| (seconds) Delay Between Injections | (RLU) Signal Observed with Standard Reagents | |
| --- | --- | --- |
| | Hybrid In Solution | PMP-Bound Hybrid |
| 0.1 | 328,000 | 2,200 |
| 10 | 339,000 | 3,350 |
| 20 | 349,000 | 3,200 |
| 30 | 345,000 | 3,250 |
| 60 | 345,000 | 3,100 |
| 150 | 350,000 | 2,900 |
| 300 | 345,000 | 2,800 |
| 600 | 382,000 | 3,000 |

## TABLE 8

| Magic-Lite Analyzer Reagents | (RLU) Reagent Background | (Moles) Hybrid Input | (RLU) Solution Chemiluminesence | (RLU) PMP-Captured Chemiluminesence |
|---|---|---|---|---|
| Standard | 370 | 5 X 10 -15 | 48,360 | 1,980 |
| Modified | 950 | 5 X 10 -15 | 78,830 | 24,920 |
| Ratio 1 | 3 | 1 | 2 | 13 |

1:   Ratio is equal to the dividend of the chemiluminescent signal observed upon activation of the MLA system with Modified Reagents divided by that observed upon activation with Standard Reagents.

**TABLE 9**

|  | CHEMILUMINESENCE | | LIQUID SCINTILLATION | |
|---|---|---|---|---|
| (fmol)<br>rRNA Input | (RLU)<br>Signal | 1<br>S/B | (CPM)<br>Signal | 2<br>S/B |
| 0.1 | 1,530 | 1.8 | 76 | 2.2 |
| 1 | 8,725 | 10.5 | 364 | 11 |
| 10 | 72,750 | 88 | 2,970 | 87 |
| 100 | 127,250 | 150 | 4,648 | 140 |
| NSB: 0.1 | 745 | | 39 | |
| 1 | 880 | | 27 | |
| 10 | 800 | $\bar{X}$ = 830 | 31 | $\bar{X}$ = 34 |
| 100 | 900 | | 39 | |
| Sensitivity 3 | 0.12 fmol | | ~0.1 fmol | |

1: Signal to Background (S/B) is the dividend of the chemiluminescent signal observed on the MLA system at a given rRNA input divided by the mean of the non-specifically bound (NSB) chemiluminescent signal observed at all rRNA inputs.

2: Signal to Background (S/B) is the dividend of the liquid scintillation signal observed at a givern rRNA input divided by the mean of the non-specifically bound (NSB) liquid scintillation signal observed at all rRNA inputs.

3: Sensitivity is defined as the amount of sandwich hybrid, in femtomoles, required to generate S/B of 2; calculated by linear interpolation.

## TABLE 10

| (fmol) rRNA Input | STANDARD REAGENTS | | MODIFIED REAGENTS | |
|---|---|---|---|---|
| | (RLU) Signal | S/B 1 | (RLU) Signal | S/B 2 |
| 0.1 | 1,795 | 1.6 | 2,445 | 1.7 |
| 0.32 | 3,005 | 2.7 | 3,680 | 2.5 |
| 1 | 4,500 | 4.1 | 7,225 | 5.0 |
| 3.2 | 18,485 | 17 | 18,885 | 13 |
| 10 | 50,455 | 46 | 53,520 | 37 |
| 0.1 | 1,030 | | 1,630 | |
| 0.32 | 1,060 | | 1,685 | |
| 1 | 1,200 $\bar{X}$ = 1,100 | | 1,185 $\bar{X}$ = 1,450 | |
| 3.2 | 1,025 | | 1,960 | |
| 10 | 1,205 | | 1,285 | |
| Sensitivity 2 | 0.18 fmol | | 0.17 fmol | |

1: Signal to Background (S/B) is the dividend of the chemiluminescent signal observed on the MLA system at a given rRNA input divided by the mean of the non-specifically bound (NSB) chemiluminescent signal observed at all rRNA inputs.

2: Sensitivity is defined as the amount of sandwich hybrid, in femtomoles, required to generate S/B of 2; calculated by linear interpolation.

## Claims

1. A method for assaying the presence of one or more target nucleic acid sequences in a test sample characterised in that it comprises: (a) providing a test sample comprising cells of one or more cell types and being suspected of containing one or more target nucleic acid sequences; (b) releasing target nucleic acid sequences from the cells thereof; (c) hybridizing the target nucleic acid sequence, if present therein, With a plurality of distinct oligonucleotide probe units to form a plurality of hybrid complexes, each of the probe units comprising at least two labelled oligonucleotide probes, each being complementary to and at

26

least one being specific to a region of the target nucleic acid sequence and at least one of the probes of the unit being labelled with one or more first support binding partners and at least one of the probes of the unit being labelled with one or more distinct detector molecules; (d) capturing the hybrid complexes on a solid support to form sandwich complexes, the solid support having one or more second support binding partners immobilized thereon which are complementary to the first support binding partners and the first support binding partners binding to the second support binding partners; (e) isolating the sandwich complexes from the test sample and non-hybridized probes of the units; and (f) detecting the presence of one or more target nucleic acid sequences by the activation of detector molecules associated with the sandwich complexes, each of the detector molecules providing a discernible activation reaction. 2. A method for assaying Campylobacter rRNA characterised in that it comprises: (a) providing a test Sample comprising cells of one or more cell types and being suspected of containing Campylobacter cells or Campylobacter rRNA, which may be of one or more species of Campylobacter ; (b) releasing rRNA from the cells thereof; (c) hybridizing rRNA of Campylobacter , if present therein, with a least two labelled oligonucleotide probes to form a hybrid complex, each of the probes having a nucleotide sequence that is complementary and at least one of which is specific to a region of Campylobacter 16S rRNA, at least one of the probes being labelled with one or more first support binding partners and at least one of the probes beang labelled with one or more detector molecules; (d) capturing the hybrid complex on a solid support to form a sandwich complex, the solid support having one or more second support binding partners immobilized thereon which are complementary to the first support binding partners and the first support binding partners binding to the second support binding partners; (e) isolating the sandwich complex from the test sample and excess non-hybridized probes; and (f) detecting the presence of Campylobacter by the activation of detector molecules associated with the sandwich complex. 3. A method as claimed in claim 1 or claim 2 wherein the first support binding partner is a hapten and the second support binding partner is an antibody; or wherein the first support binding partner is an antibody or antigen and the second support binding partner is an antigen or antibody, respectively. 4. A method as claimed in any of claims 1 to 3 wherein the oligonucleotide probes or units include at least two distinct nucleotide sequences selected from: (a) 5'-AAC TTT CCC TAC TCA ACT TGT GTT AAG CAG GAG TAT AGA GTA TTA GCA GTC G-3', or (b) 5'-TAC TCA ACT TGT GTT AAG CAG GAG TAT AGA-3', or (c) 5'-GTT AAG CAG GAG TAT AGA GTA TTA GCA GTC G-3', or (d) 5'-GTA CCG TCA GAA TTC TTC CCT AAG AAA-3', or (e) 5'-TCT GCC TCT CCC TCA CTC TAG ACT ATG AGT T-3', or (f) 5'-ACT AGC ATC CCA ACA ACT AGT GTA C-3', or (g) 5'-AAC TTT CCC TAC TCA ACT TGT-3', or (h) 5'-TCT GCC TCT CCC TCA CTC TAG ATT ATC AGT T-3', or (i) 5'-TCT GCC TCT CCC TCA CTC TAG ATT ATG AGT T-3', or (j) 5'-GGA GTA TGG AGT ATT AGC AGT CAT TTC TAA-3', or (k) 5'-ACT GCC GTG ACT AGC ACA GCA ACA AC-3', or (l) 5'-TGT TAG CAA CTA AAT ACG TGG GTT GCG-3',or (m) 5'-GCC TTC GCA ATG GGT ATT CTT GGT GAT-3'. 5. A method as claimed in any of claims 1 to 4 wherein the detector molecule is an enzyme or a chemiluminescent molecule, preferably an acridinium ester, more preferably a dimethylacridinium ester. 6. A method as claimed in any of claims 1 to 5 wherein the solid support comprises paramagnetic particles. 7. A method as claimed in any of claims 1 to 6 wherein the detector molecule is activated by the addition of a first reagent solution and then, following an incubation time, the addition of a second reagent solution, so that a detectable light reaction is initiated, the first reagent solution comprising 1.0 N $HNO_3$ in a 0.5% solution of $H_2O_2$ and the second reagent solution comprising 2.5 N NaOH in a 0.5% solution of a surfactant, preferably Arquad. 8. A method as claimed in any of claims 4 to 7 further comprising quantitating the number of bacterial cells in the test sample and the sensitivity of the test assay permitting quantitation of approximately $1 \times 10^4$ bacteria cells per milliliter of test sample. 9. A method as claimed in any of claims 1 to 8 wherein the first support binding partner comprises avidin/strepavidin or biotin and the second support binding partner comprises biotin or avidln/strepavidin, respectively; or, when the first support binding partner is a hapten and the second support binding partner is an antibody, the first support binding partner comprises dinitrophenol and the second support binding partner comprises anti-dinitrophenol antibodies. 10. A method as claimed in any of claims 1 to 9 wherein the rRNA is released from cells, not in the presence of stool, by lysis in 0. 25% SDS in Tris, EDTA buffer (pH 8-9) for 10 minutes at room temperature, and wherein, in the presence of stool, the release of rRNA from cells is by lysis in 0. 25% SDS in Tris, EDTA buffer (p/h 8-9) for 10 minutes at 75°C and then filtration. 11. A method as claimed in any of claims 1 to 10 wherein the oligonucleotide probes correspond to the region comprising: (a) bases 0163-0268 of E.coli 16S rRNA, or (b) bases 0391-0450 of E.coli 16S rRNA, or (c) bases 0631-0868 of E.coli 16S rRNA, or (d) bases 1102-1145 of E.coli 16S rRNA. 12. A test kit suitable for detecting and quantitating the presence of one or more target nucleic acid sequences in a test sample characterised in that it comprises: (a) a solution for releasing target nucleic acid sequences from the cells; (b) at least two distinct first labelled oligonucleotide probes, each of the probes being complementary to a target nucleic acid sequence and the label being first support binding partner; (c) at least two distinct second labelled oligonucleotide probes, each of the probes having a discernible label and being complementary to a target nucleic aicd sequence and each being complementary to a target nucleic acid sequence as is one of the first probes, the label being a detector molecule and at least one of either the first probes or the second probes, complementary to the same target nucleic acid sequence, being specific to a region of the target nucleic acid sequence; and (d) a labelled solid support, the label being a second support binding partner which is complementary to the first support binding partner. 13. A test kit suitable for detecting and quantitating Campylobacter in a test sample characterised in that it comprises: (a) a solution for releasing rRNA from Campylobacter cells; (b) a first labelled oligonucleotide probe, the probe being complementary to Campylobacter rRNA and the label being a first support binding partner; (c) a second labelled oligonucleotide probe, the probe being complementary to Campylobacter rRNA and the label being a detector molecule; the first probe or the second probe being specific to a region of Campylobacter 16S rRNA; (d) a solid support having a second support binding partner bound thereto, which is complementary to the first support binding partner. 14. An oligonucleotide probe characterised in that it complementary and specific for Campylobacter 16S rRNA and has a nucleotide sequence comprising or corresponding to one of those listed in claim 4 or claim 11. 15. A method as claimed in claim 1 wherein the target nucleic acid sequence comprises the 5S or 16S or 23S sub-unit of ribosomes of a genus or species of bacteria. 16. A method as claimed in claim 1 or claim 15 wherein the distinct detector molecules are activated sequentially or simultaneously. 17. A method or kit as claimed in any of claims 1 to 16 wherein each of the probes is specific and complementary to mutually exclusive regions of Campylobacter 16S rRNA or of the target nucleic acid sequence.

## Sequence Listing

SEQ ID No:           1

SEQUENCE TYPE:        Pure nucleotide sequence

SEQUENCE LENGTH:      52 bases

STRANDEDNESS:         Single

TOPOLOGY:             Linear

MOLECULE TYPE:        cDNA to rRNA

ORIGINAL SOURCE:      Synthetic

FEATURES:             Location corresponding to E.coli 16S rRNA
                      0163-0214

PROPERTIES:           Oligonucliotide probe specific and complementary
                      for Campylobacter 16S rRNA

                      AAC TTT CCC TAC TCA ACT TGT GTT AAG CAG GAG TAT
                      AGA GTA TTA GCA GTC G                         52

---

SEQ ID No:           2

SEQUENCE TYPE:        Pure nucleotide sequence

SEQUENCE LENGTH:      30 bases

STRANDEDNESS:         Single

TOPOLOGY:             Linear

MOLECULE TYPE:        cDNA to rRNA

ORIGINAL SOURCE:      Synthetic

FEATURES:             Location corresponding to E.coli 16S rRNA
                      0176-0205

PROPERTIES:           Oligonucliotide probe specific and complementary
                      for Campylobacter 16S rRNA

                      TAC TCA ACT TGT GTT AAG CAG GAG TAT AGA        30

SEQ ID No:              3

SEQUENCE TYPE:          Pure nucleotide sequence

SEQUENCE LENGTH:        31 bases

STRANDEDNESS:           Single

TOPOLOGY:               Linear

MOLECULE TYPE:          cDNA to rRNA

ORIGINAL SOURCE:        Synthetic

FEATURES:               Location corresponding to E.coli 16S rRNA
                        0163-0204

PROPERTIES:             Oligonucliotide probe specific and complementary
                        for Campylobacter 16S rRNA

                        GTT AAG CAG GAG TAT AGA GTA TTA GCA GTC G        31

---

SEQ ID No:              4

SEQUENCE TYPE:          Pure nucleotide sequence

SEQUENCE LENGTH:        27 bases

STRANDEDNESS:           Single

TOPOLOGY:               Linear

MOLECULE TYPE:          cDNA to rRNA

ORIGINAL SOURCE:        Synthetic

FEATURES:               Location corresponding to E.coli 16S rRNA
                        0437-0463

PROPERTIES:             Oligonucliotide probe specific and complementary
                        for Campylobacter 16S rRNA

                        GTA CCG TCA GAA TTC TTC CCT AAG AAA        27

SEQ ID No:            5

SEQUENCE TYPE:        Pure nucleotide sequence

SEQUENCE LENGTH:      31 bases

STRANDEDNESS:         Single

TOPOLOGY:             Linear

MOLECULE TYPE:        cDNA to rRNA

ORIGINAL SOURCE:      Synthetic

FEATURES:             Location corresponding to E.coli 16S rRNA
                      0641-0671

PROPERTIES:           Oligonucliotide probe specific and complementary
                      for Campylobacter 16S rRNA

                      TCT GCC TCT CCC TCA CTC TAG ACT ATG AGT T        31

---

SEQ ID No:            6

SEQUENCE TYPE:        Pure nucleotide sequence

SEQUENCE LENGTH:      25 bases

STRANDEDNESS:         Single

TOPOLOGY:             Linear

MOLECULE TYPE:        cDNA to rRNA

ORIGINAL SOURCE:      Synthetic

FEATURES:             Location corresponding to E.coli 16S rRNA
                      0821-0845

PROPERTIES:           Oligonucliotide probe specific and complementary
                      for Campylobacter 16S rRNA

                      ACT AGC ATC CCA ACA ACT AGT GTA C                25

| | |
|---|---|
| SEQ ID No: | 7 |
| SEQUENCE TYPE: | Pure nucleotide sequence |
| SEQUENCE LENGTH: | 21 bases |
| STRANDEDNESS: | Single |
| TOPOLOGY: | Linear |
| MOLECULE TYPE: | cDNA to rRNA |
| ORIGINAL SOURCE: | Synthetic |
| FEATURES: | Location corresponding to E.coli 16S rRNA 0195-0215 |
| PROPERTIES: | Oligonucliotide probe specific and complementary for Campylobacter 16S rRNA |

AAC TTT CCC TAC TCA ACT TGT                 21

---

| | |
|---|---|
| SEQ ID No: | 8 |
| SEQUENCE TYPE: | Pure nucleotide sequence |
| SEQUENCE LENGTH: | 31 bases |
| STRANDEDNESS: | Single |
| TOPOLOGY: | Linear |
| MOLECULE TYPE: | cDNA to rRNA |
| ORIGINAL SOURCE: | Synthetic |
| FEATURES: | Location corresponding to E.coli 16S rRNA 0641-0671 |
| PROPERTIES: | Oligonucliotide probe specific and complementary for Campylobacter 16S rRNA |

TCT GCC TCT CCC TCA CTC TAG ATT ATC AGT T     31

SEQ ID No:                 9

SEQUENCE TYPE:             Pure nucleotide sequence

SEQUENCE LENGTH:           31 bases

STRANDEDNESS:              Single

TOPOLOGY:                  Linear

MOLECULE TYPE:             cDNA to rRNA

ORIGINAL SOURCE:           Synthetic

FEATURES:                  Location corresponding to E.coli 16S rRNA
                           0641-0671

PROPERTIES:                Oligonucliotide probe specific and complementary
                           for Campylobacter 16S rRNA

                           TCT GCC TCT CCC TCA CTC TAG ATT ATG AGT T      31

---

SEQ ID No:                 10

SEQUENCE TYPE:             Pure nucleotide sequence

SEQUENCE LENGTH:           30 bases

STRANDEDNESS:              Single

TOPOLOGY:                  Linear

MOLECULE TYPE:             cDNA to rRNA

ORIGINAL SOURCE:           Synthetic

FEATURES:                  Location corresponding to E.coli 16S rRNA
                           0156-0185

PROPERTIES:                Oligonucliotide probe specific and complementary
                           for Campylobacter 16S rRNA

                           GGA GTA TGG AGT ATT AGC AGT CAT TTC TAA      30

SEQ ID No:              11

SEQUENCE TYPE:          Pure nucleotide sequence

SEQUENCE LENGTH:        26 bases

STRANDEDNESS:           Single

TOPOLOGY:               Linear

MOLECULE TYPE:          cDNA to rRNA

ORIGINAL SOURCE:        Synthetic

FEATURES:               Location corresponding to E.coli 16S rRNA
                        0829-0854

PROPERTIES:             Oligonucliotide probe specific and complementary
                        for Campylobacter 16S rRNA

                        ACT GCC GTG ACT AGC ACA GCA ACA AC          26


SEQ ID No:              12

SEQUENCE TYPE:          Pure nucleotide sequence

SEQUENCE LENGTH:        27 bases

STRANDEDNESS:           Single

TOPOLOGY:               Linear

MOLECULE TYPE:          cDNA to rRNA

ORIGINAL SOURCE:        Synthetic

FEATURES:               Location corresponding to E.coli 16S rRNA
                        1107-1140

PROPERTIES:             Oligonucliotide probe specific and complementary
                        for Campylobacter 16S rRNA

                        TGT TAG CAA CTA AAT ACG TGG GTT GCG          27

SEQ ID No:                13

SEQUENCE TYPE:            Pure nucleotide sequence

SEQUENCE LENGTH:          27 bases

STRANDEDNESS:             Single

TOPOLOGY:                 Linear

MOLECULE TYPE:            cDNA to rRNA

ORIGINAL SOURCE:          Synthetic

FEATURES:                 Location corresponding to E.coli 16S rRNA
                          0706-0732

PROPERTIES:               Oligonucliotide probe specific and complementary
                          for Campylobacter 16S rRNA

                          GCC TTC GCA ATG GGT ATT CTT GGT GAT          27

# FIG.1

# FIG.2

CAMPYLOBACTER rRNA fmoles

# FIG.3

CAMPYLOBACTER rRNA fmoles